(19) Europäisches Patentamt
European Patent Office
Office européen des brevets

(11) Veröffentlichungsnummer: **0 157 259**
**A1**

(12) **EUROPÄISCHE PATENTANMELDUNG**

(21) Anmeldenummer: **85103010.6**

(22) Anmeldetag: **15.03.85**

(51) Int. Cl.⁴: **C 07 D 249/12**, C 07 D 401/04, A 61 K 31/41

(30) Priorität: **16.03.84 CH 1330/84**

(43) Veröffentlichungstag der Anmeldung: **09.10.85 Patentblatt 85/41**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Jaeggi, Knut A., Dr., General Guisan-Strasse 44, CH-4054 Basel (CH)**

(74) Vertreter: **Zumstein, Fritz sen., Dr. et al, Bräuhausstrasse 4, D-8000 München 2 (DE)**

(54) **3-Mercapto-1,2,4-triazacycloalkadienderivate.**

(57) 3-Mercapto-1,2,4-triazacycloalkadienderivate der Formel I

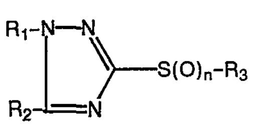

(I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/oder Trifluormethyl substituierte Aryl- oder gegebenenfalls N-oxidierte Heteroarylreste bedeuten, n für 0, 1 oder 2 steht und $R_3$ einen durch eine gegebenenfalls veresterte oder amidierte Carboxygruppe, Cyano oder in höherer als der α-Stellung durch eine oder zwei gegebenenfalls veresterte oder verätherte Hydroxygruppe(n), eine oder zwei verätherte Mercaptogruppe(n), eine oxidierte Mercaptogruppe oder eine Oxogruppe substituierten aliphatischen Kohlenwasserstoffrest bedeutet, und ihre Salze haben analgetische Eigenschaften und können als Arzneimittel für die Behandlung schmerzhaft-entzündlicher Erkrankungen verwendet werden. Sie werden hergestellt, indem man z.B. aus einer Verbindung der Formel

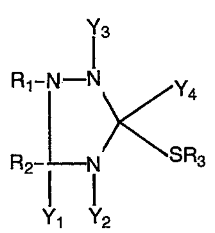

(II),

worin einer der Reste $Y_1$ und $Y_2$ einen abspaltbaren Rest Y und der andere ein Wasserstoffatom bedeutet und $Y_3$ und $Y_4$ gemeinsam eine zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzlichen Bindung HY abspaltet.

CIBA-GEIGY AG 4-14798/+
Basel (Schweiz)

3-Mercapto-1,2,4-triazacycloalkadienderivate

Die Erfindung betrifft neue 3-Mercapto-1,2,4-triazacycloalkadienderivate der Formel I

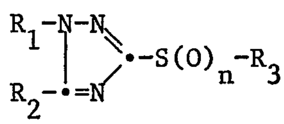
(I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/-oder Trifluormethyl substituierte Aryl- oder gegebenenfalls N-oxidierte Heteroarylreste bedeuten, n für 0, 1 oder 2 steht und $R_3$ einen durch eine gegebenenfalls veresterte oder amidierte Carboxygruppe, Cyano oder in höherer als der $\alpha$-Stellung durch eine oder zwei gegebenenfalls veresterte oder verätherte Hydroxygruppe(n), eine oder zwei verätherte Mercaptogruppe(n), eine oxidierte Mercaptogruppe oder eine Oxogruppe substituierten aliphatischen Kohlenwasserstoffrest bedeutet, und ihre Salze, Verfahren zur Herstellung der erfindungsgemässen Verbindungen, diese enthaltende pharmazeutische Präparate und ihre Verwendung.

Heteroarylreste sind beispielsweise monocyclische, ein Sauerstoff- oder Schwefelatom und gegebenenfalls zusätzlich ein Stickstoffatom aufweisende 5-gliedrige oder ein oder zwei Stickstoffatome aufweisende 6-gliedrige Heteroarylreste, wie Furyl, z.B. 2-Furyl, Thienyl,

z.B. 2-Thienyl, Thiazolyl, z.B. 2-Thiazolyl, Pyridyl, z.B. 2-, 4- oder insbesondere 3-Pyridyl bzw. 3-(1-Oxidopyridyl), oder Pyrimidyl, z.B. 2- oder 4-Pyrimidyl bzw. 2-(1-Oxidopyrimidyl).

Arylreste sind Arylreste mit bis und mit 10 Kohlenstoffatomen (C-Atomen), beispielsweise monocyclische Arylreste, wie Phenyl.

Aryl- bzw. Heteroarylreste können einen oder mehr als einen, z.B. ein, zwei oder drei, gleiche oder verschiedene der genannten Substituenten aufweisen.

Veräthertes Hydroxy ist dabei beispielsweise Niederalkoxy oder vicinal gebundenes Niederalkylendioxy bzw. Niederalkylidendioxy, kann aber auch Niederalkenyloxy oder Niederalkenyloxy sein.

Verestertes Hydroxy als Substituent von $R_1$ bzw. $R_2$ ist beispielsweise mit einer Mineralsäure oder einer organischen Carbonsäure verestertes Hydroxy, wie Halogen, Niederalkanoyloxy, ferner gegebenenfalls wie angegeben, z.B. durch Niederalkyl, Niederalkoxy, Halogen und/oder Trifluoromethyl, substituiertes Benzoyloxy.

Gegebenenfalls oxidiertes veräthertes Mercapto ist beispielsweise Niederalkylthio, Niederalkansulfonyl oder Niederalkansulfonyl.

Aliphatisch substituierte Aminosubstituenten sind beispielsweise durch Niederalkyl mono- oder disubstituiertes Amino, wie N-Mono- oder N,N-Diniederalkylamino, kann aber auch 4 bis 7-gliedriges Alkylen- bzw. 3-Aza-, 3-Oxa- oder 3-Thiaalkylenamino sein.

Verestertes Carboxy bedeutet beispielsweise aliphatisch verestertes Carboxy, wie Niederalkoxycarbonyl.

Amidiertes Carboxy ist beispielsweise unsubstituiertes oder aliphatisch substituiertes Carbamyl, wie Carbamyl, N-Mono- oder N,N-Diniederalkylcarbamyl, kann aber auch 4- bis 7-gliedriges N,N-Niederalkylen- bzw. N,N-(3-Aza-, 3-Oxa- bzw. 3-Thianiederalkylencarbamyl sein.

Aliphatische Kohlenwasserstoffreste sind beispielsweise Niederalkyl-, ferner Niederalkenyl- oder Niederalkinylreste. Als Substituenten von $R_3$, welches insbesondere substitiuertes Niederalkyl bedeutet, kommen beispielsweise gegebenenfalls mit einer organischen Carbonsäure verestertes Hydroxy, z.B. Niederalkanoyloxy, oder aliphatisch veräthertes Hydroxy, z.B. Niederalkoxy, Niederalkenyloxy, Niederalkylendioxy, gegebenenfalls oxidiertes veräthertes Mercapto, wie Niederalkylthio, Niederalkansulfinyl, Niederalkansulfonyl, Niederalkylendithio, gegebenenfalls verestertes oder amidiertes Carboxy, wie Carboxy, Niederalkoxycarbonyl, Carbamyl oder N-Mono- oder N,N-Diniederalkylcarbamyl, Cyano sowie Oxo in Betracht. Reste $R_3$ sind dementsprechend beispielsweise Carboxyniederalkyl, Niederalkoxycarbonylniederalkyl, Carbamylniederalkyl, N-Mono- und N,N-Diniederalkylcarbamylniederalkyl, Cyanoniederalkyl oder die Hydroxy-, Niederalkanoyloxy-, Niederalkoxy-, Niederalkylendioxy-, Niederalkylthio-, Niederalkylendithio-, Niederalkansulfinyl-, Niederalkansulfonyl- bzw. Oxogruppe(n) in höherer als der α-Stellung tragendes Mono- oder Dihydroxyniederalkyl, Niederalkanoyloxyniederalkyl, Mono- oder Diniederalkoxyniederalkyl, Niederalkylendioxyniederalkyl, Mono- oder Diniederalkylthio-, Niederalkansulfinyl- bzw. Niederalkansulfonylniederalkyl, Niederalkylendithioniederalkyl oder Oxoniederalkyl.

Vor- und nachstehend sind unter "niederen" organischen Resten und Verbindungen vorzugsweise solche zu verstehen, die bis und mit 7, vor allem bis und mit 4, Kohlenstoffatome (C-Atome) enthalten.

Niederalkyl ist beispielsweise Methyl, Aethyl, Propyl, Isopropyl, Butyl, Isobutyl, Sekundärbutyl oder Tertiärbutyl, ferner Pentyl, Hexyl oder Heptyl.

Niederalkenyl ist beispielsweise Vinyl, vorzugsweise jedoch über ein gesättigtes C-Atom gebundenes Niederalkenyl, wie Allyl, Methallyl oder But-2-enyl.
Niederalkinyl ist beispielsweise Aethinyl, vorzugsweise jedoch über ein gesättigtes C-Atom gebundenes Niederalkinyl, wie Propargyl oder But-2-inyl.

Niederalkoxy ist beispielsweise Methoxy, Aethoxy, Propyloxy, Isopropyloxy, Butyloxy, Isobutyloxy, Sekundärbutyloxy oder Tertiärbutyloxy, ferner Pentyloxy, Hexyloxy oder Heptyloxy. Niederalkenyloxy ist z.B. Allyloxy, Niederalkinyloxy, z.B. Propargyloxy.

Niederalkylendioxy ist beispielsweise Methylendioxy, Aethylendioxy, 1,3-Propylendioxy, 2,3-Butylendioxy oder 1,3-(2,2-Dimethyl)-propylendioxy; Niederalkylidendioxy z.B. Aethylidendioxy oder Isopropylidendioxy.

Niederalkanoyloxy ist beispielsweise Acetoxy, Propionyloxy, Butyryloxy, Isobutyryloxy, ferner Formyloxy oder Pivaloyloxy.

Niederalkylthio ist beispielsweise Methylthio, Aethylthio, Propylthio, Isopropylthio, Butylthio, Isobutylthio, Sekundärbutylthio oder Tertiärbutylthio, ferner Pentylthio, Hexylthio oder Heptylthio. Niederalkylendithio ist beispielsweise Aethylendithio, 1,3-Propylendithio oder 1,3-(2,2-Dimethyl)-propylendithio.

Niederalkansulfinyl ist beispielsweise Methan-, Aethan-, 1- oder 2-Propan-, Butan- oder Isobutansulfinyl.
Niederalkansulfonyl ist beispielsweise Methan-, Aethan-, 1- oder 2-Propan-, Butan- oder Isobutansulfonyl.

Mono- oder Diniederalkylamino ist beispielsweise Methylamino, Dimethylamino, Aethylamino, Diäthylamino, Propylamino oder Butylamino.

4- bis 7-gliedriges Alkylenamino bzw. 3-Aza-, 3-Oxa- oder 3-Thiaalkylenamino ist beispielsweise Pyrrolidino, Piperidino, Morpholino, Thiomorpholino oder Piperazino bzw. 4-Methyl- oder 4-Aethylpiperazino.

4- bis 7-gliedriges N,N-Niederalkylen- bzw. N,N-(3-Aza-, 3-Oxa- bzw. 3-Thia)-niederalkylencarbamylniederalkyl ist beispielsweise Pyrrolidino-, Piperidino-, Morpholino-, Thiomorpholino- oder Piperazino- bzw. 4-Methyl- oder 4-Aethylpiperazinocarbonylmethyl.

Carboxyniederalkyl ist beispielsweise Carboxymethyl, 1- oder 2-Carboxyäthyl, 1-, 2- oder 3-Carboxypropyl, 2-(2-Carboxy)-propyl oder 4-Carboxybutyl, ferner 2-(2-Carboxy)-butyl.

Niederalkoxycarbonylniederalkyl ist beispielsweise Methoxycarbonylmethyl, Aethoxycarbonylmethyl, 1- oder 2-Methoxycarbonyläthyl, 1- oder 2-Aethoxycarbonyläthyl, 1-, 2- oder 3-Methoxycarbonylpropyl, 1-, 2- oder 3-Aethoxycarbonylpropyl, 2-(2-Methoxycarbonyl)- bzw. 2-(2-Aethoxycarbonyl)-propyl oder 4-Aethoxycarbonyl- bzw. 4-Methoxycarbonylbutyl, ferner 2-(2-Methoxycarbonyl)- bzw. 2-(Aethoxycarbonyl)-butyl.

Carbamylniederalkyl bzw. N-Mono- oder N,N-Diniederalkylcarbamylniederalkyl ist beispielsweise Carbamylmethyl, 1- oder 2-Carbamyläthyl oder 2-(2-Carbamyl)-propyl bzw. N-Methyl-, N-Aethyl- oder N,N-Dimethylcarbamylmethyl, 1- oder 2-(N-Methyl-, N-Aethyl- oder N,N-Dimethylcarbamyl)-äthyl oder 2-[2-(N-Methyl-, N-Aethyl- oder N,N-Dimethylcarbamyl)]-propyl.

Cyanoniederalkyl ist beispielsweise Cyanomethyl, 1-Cyanoäthyl oder 2-(2-Cyano)-propyl.

Mono- oder Diniederalkoxyniederalkyl ist beispielsweise 2-Methoxy-, 2-Aethoxy-, 2-Propyloxy- oder 2-Isopropyloxy-, 2,2-Dimethoxy- oder 2,2-Diäthoxy-äthyl, 3-Methoxy- oder 3-Aethoxy-propyl oder 3,3-Dimethoxy-, 3,3-Diäthoxy-, 2,3-Dimethoxy- oder 2,3-Diäthoxypropyl, 2-(1-Dimethoxy-2-methyl)-propyl oder 4,4-Dimethoxy-butyl.

Niederalkylendioxyniederalkyl ist beispielsweise 2,2-Aethylendioxy- oder 2,2-Propylendioxy-äthyl oder 3,3- oder 2,3-Aethylendioxy-propyl, ebenso 2-(1-Aethylendioxy-2-methyl)-propyl.

Niederalkanoyloxyniederalkyl ist beispielsweise 1- oder 2-Acetoxyäthyl, 1- oder 2-Pivaloyloxyäthyl, 1-Acetoxypropyl-(2), 1-Pivaloyloxypropyl-(2), 1-Acetoxy-2-methyl-propyl-(2) oder 1-Pivaloyloxy-2-methyl-propyl-(2) oder 3-Acetoxypropyl.

Mono- oder Diniederalkylthioniederalkyl bedeutet beispielsweise 2-Methylthio-, 2-Aethylthio-, 2-Propylthio-, 2-Isopropylthio-, 2,2-Dimethylthio- oder 2,2-Diäthylthio-äthyl, 3-Methylthio- oder 3-Aethylthio-propyl oder 3,3-Bis(methylthio)-, 3,3-Bis(äthylthio)-, 2,3-Bis(methylthio)- oder 2,3-Bis(äthylthio)-propyl oder 4,4-Bis-(methylthio)-butyl.

Niederalkansulfinylniederalkyl ist beispielsweise 2-Methansulfinyl-, 3-Aethansulfinyl-, 2-Propansulfinyl- oder 2-(2-Propansulfinyl)-äthyl, 3-Methansulfinyl- oder 2-Aethansulfinyl-propyl.

Niederalkansulfonylniederalkyl ist beispielsweise 2-Methansulfonyl-, 3-Aethansulfonyl-, 2-Propansulfonyl- oder 2-(2-Propansulfonyl)-äthyl, 3-Methansulfonyl- oder 3-Aethansulfonyl-propyl.

Niederalkylendithioniederalkyl ist beispielsweise 2,2-Aethylendithio- oder 2,2-Propylendithio-äthyl oder 3,3- oder 2,3-Aethylendithiopropyl.

Mono- oder Dihydroxyniederalkyl ist beispielsweise Hydroxymethyl, 1- oder 2-Hydroxyäthyl, 3-Hydroxy- oder 2,3-Dihydroxy-propyl, 2-Hydroxypropyl-(2), 1-Hydroxy-2-methyl-propyl-(2), 4-Hydroxy- oder 2,4-Dihydroxybutyl, 5-Hydroxy-, 2,5-Dihydroxy- oder 3,5-Dihydroxypentyl.

Oxoniederalkyl bedeutet beispielsweise 2-Oxoäthyl, 2- oder 3-Oxopropyl oder 2-, 3- oder 4-Oxobutyl, ferner entsprechendes Oxopentyl, Oxohexyl oder Oxoheptyl oder Formyl.

Halogen ist beispielsweise Halogen der Atomnummer bis und mit 35, wie Fluor, Chlor oder Brom.

Salze von Verbindungen der Formel I sind vor allem pharmazeutisch verwendbare Salze, einerseits pharmazeutisch verwendbare Säureadditionssalze mit starken Säuren, wie Mineralsäure, z.B. Salze mit Halogenwasserstoffsäuren, vor allem Chlor- oder Bromwasserstoffsäure, d.h. Hydrohalogenide, vor allem Hydrochloride und Hydrobromide, oder Schwefelsäuresalze, d.h. Hydrogensulfate und Sulfate, sowie Salze mit geeigneten organischen Säuren, wie Dicarbonsäuren oder organischen Sulfonsäuren, z.B. Maleinate, Fumarate, Maleate, Tartrate oder Methansulfonate, und N-Cyclohexylsulfaminate, andrerseits innere Salze von Verbindungen der Formel I, worin $R_3$ Carboxy aufweist, oder pharmazeutisch verwendbare Metall-, wie Alkali- oder Erdalkalimetallsalze oder Ammoniumsalze mit Ammoniak oder geeigneten organischen Aminen, wie Mono-, Di- oder Triniederalkylaminen, z.B. Diäthylamin, Mono-, Di- oder Trihydroxyniederalkylaminen, z.B. Triäthanolamin oder 2-Dimethylaminoäthanol, oder heteroaliphatischen Aminen, z.B. Morpholin.

Die Verbindungen der Formel I weisen wertvolle pharmakologische Eigenschaften auf. Insbesondere zeigen sie eine ausgeprägte antinociceptive Wirksamkeit sowie eine Hemmwirkung auf die Prostaglandinsynthese, ebenso eine deutliche antiinflammatorische Wirkung. So erweisen sie sich an der Maus im durch Phenyl-p-benzochinon ausgelösten Writhing-Syndrom nach J. Pharmacol. exp. Therap. 125, 237 (1959) im Dosisbereich von etwa 3 - 50 mg/kg p.o. als ausgezeichnet wirksam.

Ebenso zeigen sie bei Dosen ab etwa 25 mg/kg p.o. eine deutliche Hemmwirkung auf das experimentelle Carrageneen-Pfotenoedem der Ratte (Di Pasquale et al: Agents and Actions 5, 256 [1975]) sowie im experimentellen Crotonölödem am Rattenohr (TONELLI et al.: Endocrinology 77, 625 [1965]) bei topischer Applikation in einer Konzentration ab etwa 20 mg/ml eine deutliche entzündungshemmende Wirkung.

Ferner zeigen sie in vitro im Konzentrationsbereich von etwa 2 bis 30 µM/L eine deutliche Hemmwirkung auf die Prostaglandinsynthese aus Arachidonsäure, nachgewiesen in der Versuchsanordnung nach Prostaglandins 7, 123 (1974).

Die Verbindungen der Formel I sind dementsprechend vorzüglich geeignet als Wirkstoffe von pharmazeutischen Präparaten zur Behandlung schmerzhaft-entzündlicher Erkrankungen, vor allem chronischer Erkrankungen des rheumatischen Formenkreises, wie der chronischen Arthritis.

Die Erfindung betrifft in erster Linie Verbindungen der Formel I, worin die Reste $R_1$ und $R_2$ unabhängig voneinander unsubstituierte oder durch Niederalkyl, Niederalkoxy bzw. an vicinalen C-Atomen Niederalkyl(id)endioxy, Hydroxy, Halogen, Niederalkanoyloxy, Niederalkylthio, Niederalkansulfinyl, Niederalkansulfonyl, Amino, Mono- oder Diniederalkylamino und/oder Trifluormethyl substituierte 6 bis und mit 10 C-Atomen aufweisende Aryl- bzw. monocyclische, ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedrige Heteroarylreste bedeuten, n für 0, 1 oder 2 steht und $R_3$ Carboxyniederalkyl, Niederalkoxycarbonylniederalkyl, Carbamylniederalkyl, N-Mono- und N,N-Diniederalkylcarbamylniederalkyl,

Cyanoniederalkyl oder die Hydroxy-, Niederalkanoyloxy-, Niederalkoxy-, Niederalkylendioxy-, Niederalkylthio-, Niederalkylendithio, Niederalkansulfinyl-, Niederalkansulfonyl- bzw. Oxogruppe(n) in höherer als der α-Stellung tragendes Mono- oder Dihydroxyniederalkyl, Niederalkanoyloxyniederalkyl, Mono- oder Diniederalkoxyniederalkyl, Niederalkylendioxyniederalkyl, Niederalkylendithioniederalkyl, Mono- oder Diniederalkylthioniederalkyl, Niederalkansulfinylniederalkyl, Niederalkansulfonylniederalkyl oder Oxoniederalkyl bedeutet, und ihre Salze.

Die Erfindung betrifft insbesondere Verbindungen der Formel I, worin die Reste $R_1$ und $R_2$ unabhängig voneinander unsubstituiertes oder durch Niederalkyl, Niederalkoxy, Hydroxy, Halogen, Niederalkanoyloxy, Niederalkylthio, Niederalkansulfonyl, Diniederalkylamino und/oder Trifluormethyl substituiertes Phenyl bzw. unsubstituiertes oder durch Niederalkyl, Niederalkoxy, Halogen oder Hydroxy substituiertes Furyl, wie 2-Furyl, Thienyl, wie 2-Thienyl, Pyridyl, wie 2-, 3- oder 4-Pyridyl, bzw. 1-Oxidopyridyl, wie 2-, 3- oder 4-(1-Oxido)-pyridyl, bedeuten, n für 0, 1 oder 2 steht und $R_3$ Carboxyniederalkyl, Niederalkoxycarbonylniederalkyl, Carbamylniederalkyl, N-Mono- oder N,N-Diniederalkylcarbamylniederalkyl oder die Hydroxy-, Niederalkoxy-, Niederalkylendioxy-, Niederalkylidendioxy- bzw. Oxogruppe(n) in höherer als der α-Stellung tragendes Hydroxyniederalkyl, Mono- oder Diniederalkoxyniederalkyl, Niederalkylendioxyniederalkyl oder Oxoniederalkyl bedeutet, und ihre Salze, insbesondere ihre pharmazeutisch verwendbaren Salze.

Die Erfindung betrifft speziell Verbindungen der Formel I, worin die Reste $R_1$ und $R_2$ unabhängig voneinander unsubstituiertes oder durch Niederalkoxy mit bis und mit 4 C-Atomen, wie Methoxy, Niederalkylthio mit bis und mit 4 C-Atomen, wie Methylthio, oder Halogen der Atomnummer bis und mit 35, wie Fluor oder Chlor, substituiertes Phenyl bedeuten,

n für 0 steht, und $R_3$ die Carboxy oder Niederalkoxycarbonylgruppe in α-Stellung tragendes Carboxy- bzw. Niederalkoxycarbonylniederalkyl mit bis und mit 4 C-Atomen im Niederalkyl- und gegebenenfalls Niederalkoxyteil,wie Carboxymethyl, 1-Carboxyäthyl, Methoxy- oder Aethoxycarbonylmethyl oder 2-(2-Carboxy)-propyl, bedeutet, und ihre, insbesondere pharmazeutisch verwendbaren, Salze.

Die Erfindung betrifft in allererster Linie Verbindungen der Formel I, worin $R_1$ und $R_2$ durch Niederalkoxy mit bis und mit 4 C-Atomen wie Methoxy, welches insbesondere in p-Stellung gebunden ist, substituierte Phenylreste bedeuten, n für 0, 1 oder 2 steht und $R_3$ die Carboxy oder Niederalkoxycarbonylgruppe in α-Stellung tragendes Carboxy- bzw. Niederalkoxycarbonylniederalkyl mit bis und mit 4 C-Atomen im Niederalkyl- und gegebenenfalls Niederalkoxyteil, wie Carboxymethyl, 1-Carboxyäthyl, Methoxy- oder Aethoxycarbonylmethyl oder 2-(2-Carboxy)-propyl,oder die Hydroxy-, Oxo-, Niederalkoxy- bzw. Niederalkylendioxygruppe in höherer als der α-Stellung tragendes Hydroxyniederalkyl mit 2 bis und mit 4 C-Atomen, wie 2-Hydroxyäthyl oder 3-Hydroxypropyl, Oxoniederalkyl mit 2 bis und mit 4 C-Atomen, wie 2-Oxoäthyl oder 3-Oxopropyl,oder Mono- oder Diniederalkoxyniederalkyl bzw. Niederalkylendioxyniederalkyl mit jeweils bis und mit 4 C-Atomen in jedem Alkyl(en)teil, wie 2-Methoxyäthyl, 2,2-Dimethoxyäthyl, 2,2-Dimethoxyäthyl, 3,3-Dimethoxypropyl oder 2,2-Aethylendioxyäthyl, bedeutet, und ihre, insbesondere pharmazeutisch verwendbaren, Salze.

Die Erfindung betrifft vor allem Verbindungen der Formel I, worin die Reste $R_1$ und $R_2$ unabhängig voneinander unsubstituiertes oder durch Niederalkoxy mit bis und mit 4 C-Atomen, wie Methoxy, Niederalkylthio mit bis und mit 4 C-Atomen, wie Methylthio, oder Halogen der Atomnummer bis und mit 35, wie Fluor oder Chlor, substituiertes Phenyl bedeuten, n für 0 steht und $R_3$ die Hydroxy-, Oxo-, Niederalkoxy- bzw. Niederalkylendioxygruppe in höherer als der α-Stellung tragendes

Hydroxyniederalkyl mit 2 bis und mit 4 C-Atomen, wie Hydroxyäthyl oder 3-Hydroxypropyl, Oxoniederalkyl mit 2 bis und mit 4 C-Atomen, wie 2-Oxoäthyl oder 3-Oxopropyl, oder Mono- oder Diniederalkoxyniederalkyl bzw. Niederalkylendioxyniederalkyl mit jeweils bis und mit 4 C-Atomen in jedem Alkyl(en)teil, wie 2-Methoxyäthyl, 2,2-Dimethoxyäthyl, 2,2-Dimethoxyäthyl, 3,3-Dimethoxypropyl oder 2,2-Aethylendioxyäthyl, bedeutet, und ihre, insbesondere pharmazeutisch verwendbaren, Salze.

Die Erfindung betrifft ganz speziell Verbindungen der Formel I, worin $R_1$ und $R_2$ durch Niederalkoxy mit bis und mit 4 C-Atomen, wie Methoxy, welches insbesondere in p-Stellung gebunden ist, substituierte Phenylreste bedeuten, n für 0, 1 oder 2 steht und $R_3$ die Carboxy oder Niederalkoxycarbonylgruppe in $\alpha$-Stellung tragendes Carboxy- bzw. Niederalkoxycarbonylniederalkyl mit bis und mit 4 C-Atomen im Niederalkyl- und gegebenenfalls Niederalkoxyteil, wie Carboxymethyl, 1-Carboxyäthyl, Methoxy- oder Aethoxycarbonylmethyl oder 2-(2-Carboxy)-propyl, bedeutet, und ihre, insbesondere pharmazeutisch verwendbaren, Salze.

Die Erfindung betrifft namentlich die in den Beispielen genannten neuen Verbindungen der Formel I und ihre pharmazeutisch verwendbaren Salze, Verfahren zu ihrer Herstellung, diese enthaltende pharmazeutische Präparate und ihre Verwendung.

Die Verbindungen der Formel I können nach an sich bekannten Methoden hergestellt werden, beispielsweise indem man aus einer Verbindung der Formel

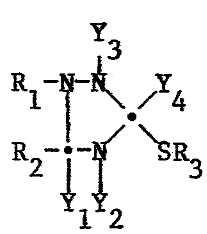

(II),

worin einer der Reste $Y_1$ und $Y_2$ einen abspaltbaren Rest Y und der andere ein Wasserstoffatom bedeutet und $Y_3$ und $Y_4$ gemeinsam eine

zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzlichen Bindung HY abspaltet, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

Abspaltbare Reste Y sind beispielsweise gegebenenfalls veresterte oder verätherte Hydroxy- oder Mercaptogruppen, ferner Amino-, Ammonio- und Sulfoniumgruppen. Als verestertes Hydroxy kommt beispielsweise mit einer anorganischen Säure oder mit einer organischen Carbonsäure verestertes Hydroxy in Betracht, wie Halogen, z.B. Chlor oder Brom, oder Niederalkanoyloxy, z.B. Acetoxy. Verätherte Hydroxygruppen sind beispielsweise Niederalkoxygruppen, z.B. Methoxy oder Aethoxy. Veresterte Mercaptogruppen sind beispielsweise mit einer Niederalkancarbonsäure veresterte Mercaptogruppen, wie Acetylthio. Verätherte Mercaptogruppen bzw. Sulfoniumgruppen sind beispielsweise Niederalkylthio- bzw. Diniederalkylsulfoniumgruppen, wie Methylthio, Aethylthio oder Dimethylsulfonium. Aminogruppen sind neben Gruppen $R_1$-NH- beispielsweise Diniederalkyl- oder Alkylen- bzw. Aza-, Oxa- oder Thia-niederalkylenaminogruppen, z.B. Dimethylamino, Diäthylamino, Pyrrolidino, Piperidino, Morpholino oder Thiomorpholino, ferner Anilino. Ammoniumgruppen sind beispielsweise der vorstehend genannten Aminogruppe entsprechende tertiäre Ammoniumgruppen oder quaternäre Ammoniumgruppen, wie Triniederalkylammonio oder Pyridinio.

Die Abspaltung von HY erfolgt in üblicher Weise spontan oder durch gelindes Erwärmen, z.B. auf etwa 40 bis 200°C, erforderlichenfalls in Gegenwart eines Hilfsmittels und/oder eines inerten Lösungsmittels. Als Hilfsmittel kommen beispielsweise saure Mittel, wie Mineralsäuren oder deren Anhydride bzw. sauren Salze, z.B. Halogenwasserstoffsäuren, vor allem Chlor-, Brom- oder Jodwasserstoffsäure, Schwefelsäure, Alkalimetallhydrogensulfate, Phosphorsäure, Polyphosphorsäure, Phosphorpentoxid, Phosphortrichlorid, Phoxphoroxychlorid oder Phosphortribromid, organische Sulfonsäuren, wie p-Toluolsulfonsäure, oder Carbonsäuren bzw. deren Anhydride, wie Niederalkansäuren und deren Anhydride oder Halogenide, z.B. Essigsäure, Acetanhydrid oder Acetylchlorid, ferner gepufferte Säurelösungen, z.B. Phosphat- oder Acetatpuffer, Hydrohalogenide von Stickstoffbasen, z.B. Ammonium- oder Pyridiniumchlorid, in Betracht.

Vielfach kann man aber auch basische Kondensationsmittel, wie Hydroxide, Carbonate oder Niederalkanolate von Alkali- oder Eralkalimetallen, z.B. Natrium-, Kalium- oder Calciumhydroxid, Natrium- oder Kaliumcarbonat oder Natriummethanolat, verwenden, ferner organische Stickstoffbasen, wie Triniederalkylamine, z.B. Tiräthylamin, oder aromatische Tertiärbasen, wie Pyridin.

Die Ausgangsstoffe der Formel II werden vorzugsweise in situ hergestellt, beispielsweise indem man eine Verbindung der Formel

$$R_A - NH - C(Y_5)(Y_6) - R_B \qquad (III)$$

worin entweder $R_A$ eine Gruppe der Formel $R_1$-NH-N=C($SR_3$)- und $R_B$ eine Gruppe $R_2$ oder $R_A$ eine Gruppe der Formel $R_2$-C(=NH)-N($R_1$)- und $R_B$ eine Gruppe -$SR_3$ darstellt und $Y_5$ und $Y_6$ unabhängig voneinander

Reste Y, wie gegebenenfalls verestertes oder veräthertes Hydroxy, veräthertes Mercapto oder gegebenenfalls substituiertes Amino bedeuten oder gemeinsam Oxo, Thioxo oder gegebenenfalls substituiertes Imino, wie Niederalkylimino, gegebenenfalls substituiertes Anilo oder Gruppen der Formel $=N-R_1$, darstellen, oder ein Salz davon cyclisiert.

Die Cyclisierung von Verbindungen der Formel III erfolgt in üblicher Weise, unter neutralen, sauren oder basischen Bedingungen, erforderlichenfalls in Gegenwart eines der genannten sauren oder basischen Mittel, in Gegenwart eines inerten Lösungs- oder Verdünnungsmittels, unter Erwärmen, z.B. im Temperaturbereich von etwa 40-200°C, vorzugsweise von etwa 60-140°C, und/oder Inertgas, wie Stickstoff.

Für die Herstellung von Verbindungen der Formel III stehen mehrere Bildungsweisen zur Verfügung. So erhält man Verbindungen der Formel III, worin $R_A$ eine Gruppe der Formel $R_1-NH-N=C(ZR_3)-$, und $R_B$ eine Gruppe $R_2$ ist und $Y_5$ und $Y_6$ gemeinsam Oxo darstellen, beispielsweise, indem man eine Verbindung der Formel $R_1-NH-N=C(SR_3)-NH_2$ (IV), die beispielsweise durch Umsetzung einer Iminoverbindung der Formel $Y-C(=NH)-SR_3$ (V), worin Y die angegebene Bedeutung hat und insbesondere Niederalkoxy, Halogen oder Amino bedeutet, bzw. eines Isothiocyanates der Formel $N{\equiv}C-SR_3$ (Va) oder eines Säureadditionssalzes, z.B. Hydrohalogenides, davon, z.B. in Aethanol, mit einem substituierten Hydrazin der Formel $R_1-NH-NH_2$ (VII) leicht zugänglich ist, oder ein Salz davon mit einer Verbindung der Formel $Y'-C(Y_5)(Y_6)-R_2$ (IX), worin $Y_5$ und $Y_6$ die angegebenen Bedeutungen haben und Y' eine Gruppe Y bzw., sofern $Y_5$ und $Y_6$ gemeinsam für Oxo stehen, eine Gruppe der Formel $R_2-C(=O)-O-$ darstellt, in üblicher Weise, z.B. in Gegenwart eines basischen Kondensationsmittels, zur Reaktion bringt.

Verbindungen der Formel III, worin $R_A$ eine Gruppe der Formel $R_2$-C(=NH)-N($R_1$)-, $Y_5$ + $Y_6$ = Oxo und $R_B$ eine $R_3$S-Gruppe ist, erhält man beispielsweise indem man eine Verbindung der Formel $R_2$-C(=NH)-Y (XVI) bzw. ein Hydrohalogenid davon mit einer Verbindung der Formel $R_1$-NH-NH-C($Y_5$)($Y_6$)-S$R_3$ (XVII), worin Y verestertes oder veräthertes Hydroxy, z.B. Halogen oder Niederalkoxy, bedeutet und $Y_5$ + $Y_6$ insbesondere für Oxo stehen, miteinander umsetzt, z.B. in einem Niederalkanol, wie Aethanol.

Im allgemeinen wird man jedoch auch die Zwischenstufe der Formel III nicht isolieren, denn die Synthese von Endstoffen der Formel I über Zwischenstufen der Formeln III und II kann gemäss einigen wichtigen Verfahrensvarianten ohne die Isolation von Zwischenprodukten durchgeführt werden. Einer wichtigen Verfahrensvariante zufolge kann man z.B. Verbindungen der Formeln $R_1$-NH-$NH_2$ (VII) und $R_2$-C($Y_5'$)($Y_6'$)-NH-C($Y_5''$)($Y_6''$)-S$R_3$ (XVIII), worin $Y_5'$, $Y_6'$, $Y_5''$ und $Y_6''$ unabhängig voneinander eine der für Y genannten Bedeutungen haben oder $Y_5'$ + $Y_6'$ und/oder $Y_5''$ + $Y_6''$ Oxo, Thioxo oder gegebenenfalls substituiertes Imino, z.B. Imino, Niederalkylimino, Anilo oder solches der Formel $R_1$-N=, darstellen, wobei Verbindungen der Formel XVIII auch in tautomerer Form, z.B. der Formeln $R_2$-C($Y_5'$)($Y_6'$)-N=C(Y)-S$R_3$ (XVIIIa) oder $R_2$-C(Y)=N-C($Y_5''$)($Y_6''$)-S$R_3$ (XVIIIb) vorliegen können, miteinander umsetzen. Die Umsetzung erfolgt in üblicher Weise, beispielsweise in Gegenwart eines der genannten sauren oder basischen Kondensationsmittels, erforderlichenfalls in Gegenwart eines inerten Lösungs- oder Verdünnungsmittels, unter Erwärmen, z.B. im Temperaturbereich von etwa 40 - 200°C, vorzugsweise von etwa 60-140°C, und/oder unter Inertgas, wie Stickstoff.

Bevorzugte Ausführungsformen dieser über intermediär gebildete Zwischenstufen der Formeln III und II verlaufenden direkten Synthesen

von Verbindungen der Formel I sind die Umsetzung von Verbindungen der Formel VII mit Verbindungen der Formel XVIII ($Y_5'$ und $Y_6'$ = Oxo, $Y_5''$ und $Y_6''$ = Thioxo)bzw. XVIIIa ($Y_5'$ und $Y_6'$ = Oxo, Y reaktionsfähiges verestertes Hydroxy, z.B. Halogen, oder veräthertes Hydroxy, z.B. Niederalkoxy, oder Diniederalkylamino) unter schwach sauren Bedingungen, z.B. in Gegenwart einer organischen Carbonsäure, wie Essigsäure, eines Ammonium-Mineralsäuresalzes, z.B. von Pyridiniumchlorid in Essigsäure, oder eines sauren Puffersystems, wie eines Acetat- oder Phosphatpuffers, die Kondensation von Verbindungen der Formeln XVI und XVII, z.B. in Aethanol bei etwa 10 - 50°C, und anschliessender Cyclisierung bei etwa 80 - 160°C, z.B. in siedendem Xylol.

Ebenfalls über Zwischenstufen der Formeln II und III verläuft die Bildungsweise von 1-$R_1$-$R_3$-5-$R_2$-1H-1,2,4-triazolen durch Umsetzung von acylierten Thiosemicarbaziden der Formel $R_2$-C(=O)-NH-C(=S)-NH-NH-$R_1$ (XXIII) mit Verbindungen der Formel $R_3$-Y (XXIV), worin $R_3$ die angegebene Bedeutung hat und Y reaktionsfähiges verestertes Hydroxy bedeutet, oder mit aliphatischen Epoxiden.

Die Verbindungen der Formel I können ferner hergestellt werden, indem man Verbindungen der Formel

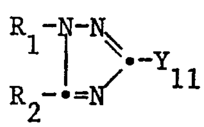 (XXXI) und $Y_{12} - R_3$ (XXXII),

worin $Y_{11}$ eine gegebenenfalls in Salzform vorliegende Mercaptogruppe und $Y_{12}$ reaktionsfähiges verestertes Hydroxy bedeutet oder $Y_{11}$ Sulfonyl und $Y_{12}$ eine gegebenenfalls in Salzform vorliegende

Mercaptogruppe darstellt, miteinander zur entsprechenden Verbindung der Formel I, worin n für 0 steht, kondensiert und erforderlichen- bzw. gewünschtenfalls eine oder mehrere der genannten Zusatzreaktionen durchführt.

Sulfonyl ist beispielsweise Niederalkansulfonyl, z.B. Methan-, oder Aethansulfonyl, oder gegebenenfalls substituiertes Benzolsulfonyl, z.B. Benzol-, p-Toluol- oder p-Brombenzolsulfonyl, ferner Fluorsulfonyl.

Als Salzform der Mercaptogruppe kommen beispielsweise deren Metallsalzformen, wie Alkalimetall- oder Erdalkalimetallsalzformen, sofern n 1 oder 2 ist, ferner deren Ammoniumsalzformen mit Ammoniak oder organischen Aminen in Betracht. Derartige Salze werden vorzugsweise durch Einwirkung der äquivalenten Menge der betreffenden Base auf die freie Mercapto-Reaktionskomponente in situ erzeugt und ohne Isolierung verwendet.

Die Kondensation erfolgt in üblicher Weise, vorzugsweise in einem inerten Lösungsmittel, erforderlichenfalls unter Kühlen oder Erwärmen, z.B. im Temperaturbereich von etwa 0 bis 100°C, in einem geschlossenen Gefäss und/oder unter Inertgas, wie Stickstoff. Als inerte Lösungsmittel kommen insbesondere polare Lösungsmittel in Betracht, wie Niederalkanole, Diniederalkylketone, N,N-Diniederalkylalkancarbonsäureamide bzw. N-Niederalkyl-lactame oder Diniederalkylsulfoxide, in Betracht. Erforderlichenfalls arbeitet man mit Katalysatoren, sofern $Y_{12}$ veräthertes Hydroxy ist, z.B. in Gegenwart von Dilithiumpalladiumtetrachlorid.

Die Ausgangsstoffe der Formel XXXI können nach an sich bekannten Methoden hergestellt werden.

So stellt man Verbindungen der Formel XXXI, worin $Y_{11}$ Mercapto bedeutet, können z.B. erhalten werden, indem man eine Verbindung der Formel $R_1$-NH-NH-C(=S)-NH-C(=O)-$R_2$ (XXXIV) zugänglich beispielsweise durch Umsetzung eines Thiosemicarbazides der Formel $R_1$-NH-NH-C(=S)-$NH_2$ (XXXV) mit einer Verbindung der Formel $R_2$-C(=O)-Y (IXb, Y = Halogen oder $R_2$-C(=O)-O-), in üblicher Weise cyclisiert. Verbindungen der Formel XXXI, worin $Y_{11}$ Mercapto ist, werden ferner erhalten, indem man eine Verbindung der Formel $R_1$-NH-$NH_2$ (VII) mit Thiophosgen oder einem Alkalirohodanid und anschliessend mit einer Verbindung der Formel IXb umsetzt.

Die Verbindungen der Formel I können ferner hergestellt werden, indem man Verbindungen der Formeln

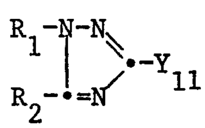 (XXXI) und $Y_{12}-R_3$ (XXXII),

worin einer der Reste $Y_{11}$ und $Y_{12}$ einen metallischen Rest und der andere eine Gruppe -S(O)$_n$-$Y_{13}$ bedeutet, in der $Y_{13}$ Halogen darstellt, miteinander kondensiert und erforderlichen- bzw. gewünschtenfalls eine oder mehrere der genannten Zusatzreaktionen durchführt.

$R_3$ bedeutet dabei einen durch verätherte Hydroxy- und/oder Mercaptogruppen substituierten aliphatischen Kohlenwasserstoffrest.

Metallische Reste sind beispielsweise Gruppen der Formeln $-M^{I}$, $-M^{II}/2$ oder $-M^{II}$-Hal, worin $M^{I}$ ein Metallatom der Gruppe 1A, z.B. Lithium oder Natrium, und $M^{II}$ ein Metallatom der Gruppen 2A und 2B des Periodischen Systems der Elemente, z.B. Magnesium, Cadmium oder Zink, bedeutet und Hal für ein Halogenatom, steht.

Halogen ist beispielsweise Chlor, Brom oder Jod.

Die Umsetzung erfolgt in üblicher Weise, vorzugsweise in einem inerten Lösungsmittel, erforderlichenfalls unter Kühlen oder Erwärmen und/oder unter Inertgas, wie Stickstoff beispielsweise im Temperaturbereich von -80 bis etwa +60°C, vorzugsweise von etwa -25 bis etwa +40°C. In einer bevorzugten Ausführungsform dieses Verfahrens geht man beispielsweise von einer Verbindung der Formel XXXI aus, worin $Y_{11}$ eine Halogensulfenylgruppe ist, und setzt diese bei etwa -10 bis +10°C in Tetrahydrofuran oder Hexamethylphosphorsäuretriamid mit einer Verbindung der Formel XXXII um, worin $Y_{12}$ ein Alkalimetallatom oder eine Halogen-Erdalkaligruppe bedeutet. In einer anderen bevorzugten Ausführungsform setzt man beispielsweise eine Verbindung der Formel XXXII, worin $Y_{11}$ ein Alkalimetallatom bedeutet, mit einer Verbindung der Formel $R_3$-S-S-$R_3$ (XXXII) um.

Ausgangsstoffe der Formel XXXI, worin $Y_{11}$ eine Gruppe der Formel -S-$Y_{13}$ und $Y_{13}$ Halogen ist, erhält man beispielsweise indem man eine Verbindung der Formel XXXI, worin $Y_{11}$ Mercapto ist, in üblicher Weise halogeniert, beispielsweise durch Umsetzung mit Chlor, z.B. in Tetrachlormethan, gegebenenfalls nach vorhergehender Behandlung mit Jod.

Verbindungen der Formel XXXI, worin $Y_{11}$ einen metallischen Rest bedeutet, können beispielsweise hergestellt werden, indem man eine Verbindung der Formel XXXI, worin $Y_{11}$ Wasserstoff bedeutet, die beispielsweise durch Umsetzung von Verbindungen der Formeln $R_2$-C(=O)-NH-CHO (XXXVI) und $R_1$-NH-$NH_2$ (VII) zugänglich ist, mit einer metallorganischen Verbindung, wie einer Alkalimetall- oder Erdalkalimetallkohlenwasserstoffverbindung, z.B. mit Butyllithium, Phenylnatrium oder Butylmagnesiumbromid, umsetzt. Man kann aber auch von einer Verbindung der Formel XXXI, worin $Y_{11}$ Halogen ist, ausgehend, und diese mit einem Erdalkalimetall, z.B. Magnesium umsetzen.

Ein weiteres Verfahren zur Herstellung von Verbindungen der Formel I ist dadurch gekennzeichnet, dass man eine Verbindung der Formel

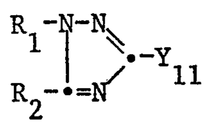

(XXXI),

worin $Y_{11}$ Mercapto ist, oder ein Salz davon mit einem von einer Verbindung der Formel $R_3$-H (XXXVII) abgeleiteten vicinalen Epoxiderivat zu einer Verbindung der Formel I umsetzt, worin $R_3$ in β-Stellung eine Hydroxygruppe aufweist und n 0 ist, und erforderlichen- bzw. gewünschtenfalls eine oder mehrere der genannten Zusatzreaktionen durchführt.

Als Salze von Verbindungen der Formel XXXI kommen beispielsweise deren Metallsalze, vorzugsweise Alkalimetall- oder Erdalkalimetallsalze in Betracht.

Von Verbindungen der Formel XXXVII abgeleitete vicinale Epoxide sind beispielsweise vicinale aliphatische Epoxide, wie α,β-Epoxyniederalkane.

Die Umsetzung erfolgt in üblicher Weise, beispielsweise in einem inerten Lösungsmittel, wie einem Niederalkanol, oder einem tertiären Amid, z.B. in Hexamethylphosphorsäuretriamid, erforderlichenfalls in Gegenwart eines Kondensationsmittels, beispielsweise ausgehend

von freien Mercaptanen der Formel XXXI eines zur Salzbildung befähigten basischen Mittels, wie eines Alkalimetallalkoholates oder -hydroxides, z.B. von Natriummethanolat oder Natriumhydroxid, unter Kühlen oder Erwärmen und/oder unter Inertgas, wie Stickstoff.

Ein weiteres Verfahren zur Herstellung von Verbindungen der Formel I ist dadurch gekennzeichnet, dass man eine Verbindung der Formel

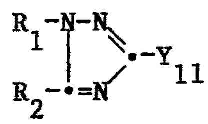

(XXXI),

worin $Y_{11}$ Mercapto ist, oder ein Salz davon mit einer Verbindung der Formel $R_3'$-H (XXXVIII), worin $R_3'$ ein von $R_3$ abgeleiteter, mindestens in $\alpha,\beta$-Stellung eine C-C-Doppel- oder C-C-Dreifachbindung aufweisenden aliphatischen Rest bedeutet, zu einer Verbindung der Formel I umsetzt, worin $R_3$ $\alpha,\beta$-gesättigt oder $\alpha,\beta$-einfach ungesättigt ist und n 0 ist, und erforderlichen- bzw. gewünschtenfalls eine oder mehrere der genannten Zusatzreaktionen durchführt.

Als Salze von Mercaptanen der Formel XXXI kommen insbesondere deren Metallsalze, vorzugsweise Alkalimetall- oder Erdalkalimetallsalze in Betracht.

Als Verbindungen der Formel XXXVIII kommen beispielsweise in Betracht: Carboxyniederalkene, Niederalkoxycarbonylniederalkene, Carbamyl- bzw. N-Niederalkyl-, N,N-Diniederalkyl-, N,N-Niederalkylen- bzw. N,N-(3-Aza-, 3-Oxa-oder 3-Thia)-niederalkylencarbamylniederalkene, die Niederalkoxy-, Niederalkylendioxy-, Niederalkylthio-, Niederalkansulfinyl-, Niederalkansulfonyl-, Hydroxy-, Niederalkanoyloxy-, Niederalkylendithio- bzw. Oxogruppe(n) in höherer als der $\beta$-Stellung tragendes Mono- oder Diniederalkoxyniederalkene, Niederalkylendioxyniederalkene, Niederalkanoyloxyniederalkene, Mono- oder Diniederalkylthioniederalkene, Niederalkansulfinyl- bzw. Niederalkansulfonylniederalkene, Niederalkylendithioniederalkene, Mono- oder Dihydroxyniederalkene und Oxoniederalkene.

Die Umsetzung erfolgt in üblicher Weise, beispielsweise in einem inerten Lösungsmittel, wie einem tertiären Amid, z.B. in Dimethylformamid oder N-Methylpyrrolidon, erforderlichenfalls in Gegenwart eines Kondensationsmittels, unter Kühlen oder Erwärmen und/oder unter Inertgas, wie Stickstoff. Als Kondensationsmittel kommen ausgehend von Mercaptanen der Formel XVI beispielsweise organische Stickstoffbasen, wie Di- oder Triniederalkylamine, z.B. Diisopropylamin oder Triäthylamin, bzw. Alkylen- bzw. Aza-, Oxa- oder Thiaalkylenamine, z.B. Pyrrolidin, Piperidin, Morpholin, Thiomorpholin oder Piperazin, in Betracht.

Die neuen Verbindungen der Formel I können ferner hergestellt werden, indem man in einer Verbindung der Formel

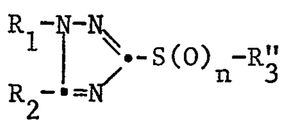

(IXL),

worin $R_3''$ einen in eine Gruppe $R_3$ überführbaren Rest bedeutet, $R_3''$ in die gewünschte Gruppe $R_3$ überführt und erforderlichen- bzw. gewünschtenfalls eine oder mehrere der genannten Zusatzreaktionen durchführt.

In Reste $R_3$ überführbare Reste sind beispielsweise durch eine gegebenenfalls zusätzliche Carboxygruppe substituierte Reste $R_3$, insbesondere 1,1-Dicarboxyniederalkyl- oder 1-Carboxy-2-oxo-niederalkylreste oder Reste der Formel $-C(=O)-O-R_3$. Die Ueberführung dieser Reste in Reste $R_3$ erfolgt durch Abspaltung von Kohlendioxid.

Die Abspaltung von Kohlendioxid kann in üblicher Weise erfolgen, beispielsweise durch Säureeinwirkung, wie Behandlung mit einer Protonensäure, wie einer Mineralsäure, z.B. von Chlorwasserstoff- oder Schwefelsäure, vorteilhaft in einem Lösungs- oder Verdünnungsmittel, erforderlichenfalls unter Erwärmen, z.B. auf etwa 50 bis etwa 250°C.

So kann man Verbindungen der Formel IXL, worin $R_3''$ einen 1-Carboxy-2-oxo- oder 1,1-Dicarboxyniederalkylrest bedeutet, durch Erwärmen mit wässrigen Säurelösungen, z.B. mit gleichen Teilen etwa 15%-iger Salzsäure und Essigsäure auf etwa 60-100°C, in Verbindungen der Formel I überführen, worin $R_3$ einen 1-Carboxy- oder 2-Oxoniederalkylrest bedeutet.

Ferner kann man Verbindungen der Formel IXL, worin $R_3''$ einen Rest der Formel $-C(=O)-O-R_3$ darstellt, z.B. durch Erwärmen mit einer Mineralsäure, z.B. mit Chlorwasserstoff in Tetrahydrofuran, unter Kohlendioxidabspaltung in die entsprechenden Verbindungen der Formel I überführen.

Weitere in Gruppen $R_3$ überführbare Reste $R_3''$ sind beispielsweise mindestens eine mit einem α-Phenylniederalkanol verätherte Hydroxygruppe bzw. veresterte Carboxygruppe oder mindestens eine mit einer Carbonsäure, wie einer halogenierten Niederalkansäure oder der Kohlensäure bzw. einem Halbester oder Halbamid derselben veresterte Hydroxygruppen, wie gegebenenfalls substituiertes Benzyloxy, gegebenenfalls halogniertes Niederalkanoyloxy oder Niederalkoxycarbonyloxy, Benzyloxycarbonyloxy, Diniederalkylcarbamyloxy oder Carbonyldioxy, aufweisende Reste $R_3''$. Als solche kommen beispielsweise in Betracht: die Benzyloxy-, Benzyloxycarbonyloxy-, die gegebenenfalls halogenierte Niederalkanoyloxy- oder Niederalkoxycarbonyloxygruppe(n) bzw. die Carbonyldioxygruppe in höherer als der α-Stellung tragendes, gegebenenfalls substituiertes Mono- oder Dibenzyloxyniederalkyl, gegebenenfalls halogeniertes Mono- oder Diniederalkanoyloxyniederalkyl

bzw. Mono- oder Diniederalkoxycarbonyloxy- oder Carbonyldioxyniederalkyl, sowie Benzyloxycarbonylniederalkylreste. Die Ueberführung derselben in $R_3$ erfolgt beispielsweise reduktiv durch Behandeln mit einem geeigneten Reduktionsmittel.

Als Reduktionsmittel kommen zur Reduktion von $\alpha$-Phenylniederalkoxy und $\alpha$-Phenylalkoxygruppen beispielsweise Wasserstoff in Gegenwart eines Hybrid, wobei man vorteilhaft in einem inerten Lösungsmittel, erforderlichenfalls bei erhöhtem Druck und/oder unter Kühlen oder Erwärmen, arbeitet. Mit Carbonsäuren veresterte Hydroxygruppen, auch $\alpha$-Phenylniederalkoxycarbonyloxygruppen, können beispielsweise mittels Dileichtmetallhydriden, z.B. mit Lithiumaluminiumhydrid, reduziert werden.

2-Halogen-niederalkoxycarbonyloxy z.B. 2,2,2-Trichlor oder 2-Jodäthoxycarbonyloxy, Aroylmethoxycarbonyloxy oder 4-Nitrobenzyloxycarbonyloxy kann z.B. durch Behandeln mit einem geeigneten chemischen Reduktionsmittel, wie Zink in Gegenwart einer geeigneten Carbonsäure, wie wässriger Essigsäure, abgespalten werden. Aroylmethoxycarbonyloxy kann auch durch Behandeln mit einem nucleophilen, vorzugsweise salzbildenden Reagens, wie Natriumthiophenolat, und 4-Nitro-benzyloxycarbonyloxy auch durch Behandeln mit einem Alkalimetall-, z.B. Natriumdithionit, gespalten werden.

Weitere Reste $R_3''$ sind zu einer Gruppe $R_3$ solvolysierbare Reste, beispielsweise Reste $R_3''$, die 1 oder 2 Halogenatom(e), eine bivalente funktionell abgewandelte Oxogruppe, z.B. Imino, eine von verestertem und amidiertem Carboxy gemäss der eingangs gegebenen Definition verschiedene funktionell abgewandelte Carboxygruppe, wie eine Iminoäthergruppierung, eine gegebenenfalls wie für amidiertes

Carboxy angegebene substituierte Amidinogruppierung, Halogencarbonyl oder gegebenenfalls substituiertes Phenoxycarbonyl, z.B. Phenoxy-, p-Nitrophenoxy- oder 2,4-Dinitrophenoxycarbonyl, oder mindestens eine von einer durch Halogen oder Aryl substituierten Alkancarbonsäure oder einem Kohlensäurehalbester abgeleitete Acyloxygruppe, mindestens eine Silyloxygruppe oder eine Arylendioxy-, wie 1,2-Phenylendioxygruppe, zwei geminal gebundene Aryloxy-, wie Phenyloxygruppen oder eine vicinal gebundene Niederalkyliden- bzw. Mono- oder Diarylniederalkylidendioxygruppe aufweisen.

Abspaltbare Acyloxygruppen sind beispielsweise Halogenniederalkanoyloxy, wie 2-Halogenacetoxy, in 1-Stellung des Niederalkylrestes verzweigtes oder 1- oder 2-Stellung geeignet substituiertes Niederalkoxycarbonyloxy, insbesondere tert.-Niederalkoxycarbonyloxy, Benzyloxycarbonyloxy das gegebenenfalls durch Niederalkyl, insbesondere tert.-Butyl, Niederalkoxy, Hydroxy, Halogen und/oder Nitro substituiert sein kann oder gegebenenfalls entsprechend substituiertes Diphenylmethoxycarbonyloxy. Solche Reste sind 2-Chlor-, 2-Brom-, 2-Jod-, 2,2,2-Trifluor- oder 2,2,2-Trichloracetoxy, tert.-Butyloxycarbonyloxy, 4-Nitrobenzyloxycarbonyloxy, Phenacycloxycarbonyloxy, 2-Halogen-niederalkoxycarbonyloxy, z.B. 2,2,2-Trichloräthoxycarbonyloxy, 2-Bromäthoxycarbonyloxy oder 2-Jodäthoxycarbonyloxy, oder 2-(trisubstituiertes Silyl)-äthoxycarbonyloxy, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyläthoxycarbonyloxy, wie 2-Trimethylsilyläthoxycarbonyloxy oder 2-(Di-n-butyl-methyl-silyl)-äthoxycarbonyloxy, oder 2-Triarylsilyläthoxycarbonyloxy, wie 2-Triphenylsilyläthoxycarbonyloxy.

Eine Silyloxygruppe ist in erster Linie Triniederalkylsilyloxy, insbesondere Trimethylsilyloxy, ferner Dimethyl-tert.-butylsilyloxy.

Ein Halogenatom, mindestens eine der ganannten Acyloxygruppen, mindestens eine Silyloxygruppe, eine Arylendioxygruppe, zwei geminal gebundene Aryloxygruppen oder eine vicinal gebundene Niederalkyliden- bzw. Mono- oder Diarylniederalkylidendioxygruppe aufweisende Reste $R_3''$ können solvolytisch in Hydroxy aufweisende aliphatische Reste $R_3$, wie Mono- oder Dihydroxyniederalkyl, ein Halogenatom aufweisende Reste $R_3'$ ferner in durch veräthertes oder organisch verestertes Hydroxy oder verätherte Mercapto substituierte Reste $R_3$, wie Niederalkoxyniederalkyl, Niederalkanoyloxyniederalkyl oder Niederalkylthioniederalkyl, zwei Halogenatome oder eine funktionell abgewandelte Oxogruppe aufweisende Reste $R_3''$ solvolytisch in Oxo aufweisende oder durch veräthertes oder organisch verestertes Hydroxy substituierte Reste $R_3$, wie Oxoniederalkyl, Diniederalkoxyniederalkyl oder Niederalkylendioxyniederalkyl, und eine funktionell abgewandelte Carboxygruppe aufeweisende Reste $R_3''$ solvolytisch in gegebenenfalls verestertes oder amidiertes Carboxy $R_3$, wie Carboxyniederalkyl, Niederalkoxycarbonylniederalkyl oder gegebenenfalls aliphatisch N-substituiertes Carbamylniederalkyl überführt werden.

Die Solvolyse der zu Hydroxy solvolysierbaren Gruppen aufweisende Reste $R_3''$ erfolgt beispielsweise durch Hydrolyse (Behandlung mit Wasser), Alkoholyse (Behandlung mit einem Alkohol), Mercaptolyse (Umsetzung mit einem Mercaptan), Umsetzung mit einer organischen Carbonsäure bzw. einem Salz davon oder Ammono- bzw. Aminolyse (Behandlung mit Ammoniak oder einem organischen Amin).

Durch Hydrolyse kann man beispielsweise ein Halogenatom, mindestens eine der genannten Acyloxygruppen, mindestens eine Silyloxygruppe, eine Arylendioxygruppe, zwei geminal gebundene Aryloxygruppen oder eine vicinal gebundene Niederalkyliden- bzw. Mono- oder Diaryl-niederalkylidendioxygruppe aufweisende Reste $R_3''$ in durch Hydroxy substituierte Reste $R_3$, wie Mono- oder Dihydroxyniederalkyl, zwei Halogenatome oder eine bivalente funktionell abgewandelte Oxogruppe aufweisende Reste $R_3''$ in Oxo aufweisende Reste $R_3$, wie Oxoniederalkyl, funktionell abgewandeltes Carboxyniederalkyl $R_3''$ in Carboxyniederalkyl sowie eine Iminoäthergruppierung, eine gegebenenfalls aliphatische N-substituierte Amidingruppierung aufweisende funktionell abgewandelte Carboxyniederalkylreste $R_3''$ in verestertes oder amidiertes Carboxyniederalkyl $R_3$ überführen.

Die Hydrolyse wird in üblicher Weise durchgeführt, erforderlichenfalls in Gegenwart eines Hydrolysemittels, und/oder eines inerten Lösungsmittels, unter Kühlen oder Erwärmen und/oder unter Inertgas. Hydrolysemittel sind beispielsweise saure oder alkalische Mittel. Saure Mittel sind beispielsweise wie Mineralsäuren, Halogenwasserstoffsäuren, z.B. Chlor-, Brom- oder Jodwasserstoffsäure, oder Sauerstoffsäuren des Schwefels oder Phosphors bzw. deren saure Salze, wie Schwefelsäure, Kaliumhydrogensulfat oder Phosphorsäure, oder organische Carbon- oder Sulfonsäuren, z.B. Niederalkansäure, wie Essigsäure, Trifluoressigsäure oder Chloressigsäure, oder p-Toluolsulfonsäure. Basische Mittel sind beispielsweise Hydroxide oder Carbonate von Alkali- oder Erdalkalimetallen, wie Kalium-, Natrium- oder Calciumhydroxid, bzw. Kalium- oder Natriumcarbonat. Inerte Lösungsmittel sind beispielsweise polare, mit Wasser mischbare Lösungsmittel, wie Alkohole, z.B. Methanol oder Aethanol, Niederalkylenäther, wie Dioxan, Diniederalkylketone, wie Aceton, tertiäre Amide, z.B. Dimethylformamid, N-Methylpyrrolidon, oder Hexamethylphosphorsäuretriamid, oder Diniederalkylsulfoxide, z.B. Dimethylsulfoxid.

Durch Alkoholyse können beispielsweise ein oder zwei Halogenatom(e) oder bivalentes funktionell abgewandeltes Oxo aufweisende aliphatische Reste $R_3''$ in durch veräthertes Hydroxy substituierte aliphatische Kohlenwasserstoffreste $R_3$, wie Mono- oder Diniederalkoxyniederalkyl, Niederalkylendioxyniederalkyl
oder Halogencarbonyl aufweisende aliphatische Reste $R_3''$ in verestertes Carboxy aufweisende Reste $R_3$, wie Niederalkoxycarbonyl, überführt und in Resten $R_3''$ mit organischen Carbonsäure veresterte Hydroygruppen durch Umesterung freigesetzt werden. Die Alkoholyse erfolgt in üblicher Weise, beispielsweise in Gegenwart eines basischen bei der Umesterung auch eines sauren Mittels, erforderlichenfalls unter Kühlen oder Erwärmen und/oder unter Inertgas, wie Stickstoff. Als saure bzw. basische Mittel kommen z.B. die genannten in Betracht, als basische Mittel ferner Metallalkoholate, wie Alkalimetall- oder Erdalkalimetallniederalkanolate, z.B. Natriummethanolat.

Durch Mercaptolyse kann man beispielsweise ein oder zwei Halogenatom(e) oder bivalentes funktionell abgewandeltes Oxo aufweisende Reste $R_3''$ in durch veräthertes Mercapto substituierte Reste $R_3$, wie Niederalkylthioniederalkyl oder Niederalkylendithioniederalkyl, überführen. Die Mercaptolyse erfolgt in üblicher Weise, z.B. in Gegenwart eines basischen Kondensationsmittels oder indem man die Mercaptankomponente in Salzform, wie als Alkalimetallsalz, einsetzt.

In einer Abwandlung dieses Verfahrens kann man auch einen mercaptosubstituierten Niederalkylrest $R_3''$ durch Umsetzung mit allen reaktiven Ester eines Niederalkanols, wie einem Niederalkylhalogenid oder Dimethylsulfat, in Niederalkylthioniederalkyl überführen.

Ein Halogenatom aufweisende aliphatische Reste $R_3''$ können auch durch Umsetzung mit einem Salz in einer Carbonsäure, wie einem Niederalkansäurealkalimetallsalz, in organisch verestertes Hydroxy aufweisende Reste $R_3$, wie Niederalkanoyloxyniederalkyl, überführt werden.

Durch Amino- bzw. Aminolyse kann man beispielsweise Halogencarbonyl aufweisende aliphatischer Reste $R_3''$ in durch amidiertes Carboxy substituierte aliphatische Kohlenwasserstoffreste $R_3$, wie gegebenenfalls aliphatisch N-substituierte Carbamylniederalkylreste, überführen, vorteilhaft in Gegenwart eines basischen Kondensationsmittels. Man kann aber durch Aminolyse auch aus den genannten Acyloxygruppen die Hydroxygruppe freisetzen.

Halogenniederalkyl kann aber auch durch Umsetzung mit Thioharnstoff zunächst in das Isothiouroniumderivat überführt und anschliessend durch Hydrolyse und Alkylierung in Niederalkylthioniederalkyl überführt werden. 2-Substituiertes Silyläthoxycarbonyloxy lässt sich vorteilhaft durch Behandeln mit einem Alkalimetallfluorid, z.B. mit Kaliumfluorid, spalten.

Die solvolytische Ueberführung von Halogen aufweisenden Resten $R_3''$ in die genannten, von Mono- oder Dihydroxyniederalkyl verschiedenen Reste $R_3$ erfolgt üblicherweise in Gegenwart eines basischen Kondensationsmittels, wie eines Alkalimetallhydroxides, oder einer tertiären organischen Stickstoffbase, wie eines Triniederalkylamins oder eine heteroaromatischen Base, wie von Pyridin, oder indem man die dem einzuführenden Rest entsprechende Komponente in Form eines Salzes, wie eines Alkali- oder Erdalkalimetallsalzes einsetzt.

Die Ausgangsstoffe können nach an sich bekannten Methoden hergestellt werden, beispielsweise durch Abspaltung von HY aus Verbindungen der Formel

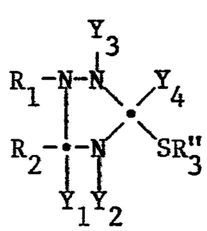

(XL),

worin $Y_1$, $Y_2$, $Y_3$ und $Y_4$ die unter der Formel II angegebenen Bedeutungen haben, und gewünschtenfalls Oxidation der Thiogruppe oder durch Umsetzung von Verbindungen der Formeln

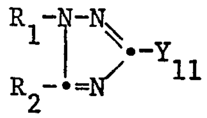 (XXXI) und $Y_{12}-R_3''$ (XLI),

worin einer der Reste $Y_{11}$ und $Y_{12}$ eine reaktionsfähige veresterte Hydroxygruppe und der andere eine in Salzform vorliegende Mercaptogruppe bedeutet.

Verfahrensgemäss oder nach anderen vorstehend nicht erwähnten Verfahrensweisen erhältiche Verbindungen der Formel I können gewünschtenfalls in an sich bekannter Weise in andere Verbindungen der Formel I umgewandelt werden. Erforderlichenfalls kann ein verfahrensgemäss erhältliches Isomerengemisch in die reinen Isomeren aufgetrennt und gewünschtenfalls eine verfahrensgemäss erhältliche Verbindung in eine andere Verbindung der Formel I überführt und/oder eine verfahrensgemäss erhältliche freie Verbindung in ein Salz oder ein verfahrensgemäss erhältliches Salz in die freie Verbindung überführt werden.

So kann man beispielsweise gegebenenfalls verätherte oder veresterte Hydroxygruppen ineinander überführen.

Weist beispielsweise mindestens einer der Reste $R_1$, $R_2$ und $R_3$ eine Hydroxygruppe auf, so lässt sich diese auf üblichem Wege veräthern. So kann man Hydroxy als Bestandteil von $R_3$ mit einem Niederalkylierungsmittel, wie einem Niederalkanol, z.B. Methanol, in Gegenwart einer Säure, wie einer Mineralsäure, z.B. Schwefelsäure, oder eines Dehydratisierungsmittels, wie Dicyclohexylcarbodiimid, und Hydroxy an einem Rest $R_1$ bzw. $R_2$ z.B. in Gegenwart von Basen, wie Alkalimetallhydroxiden oder -carbonaten, z.B. Natriumhydroxid oder Kaliumcarbonat, mit Hilfe von Diniederalkylsulfaten oder Diazoniederalkanen

in entsprechende Niederalkoxygruppen überführt. Umgekehrt lassen sich Aether, wie Niederalkoxygruppen als Substituenten von $R_1$ bzw. $R_2$ abspalten, beispielsweise durch Behandeln mit Säuren, wie mit Lewissäuren, z.B. Bortribromid oder Mineralsäuren, z.B. von Jodwasserstoff.

Weiterhin lässt sich Hydroxy verestern, z.B. in Niederalkanoyloxy umwandeln, beispielsweise durch Umsetzung mit einer entsprechenden Niederalkancarbonsäure, wie Essigsäure, oder einem reaktionsfähigen Derivat, wie einem symmetrischen Anhydrid, davon oder einem Anhydrid mit einer Halogenwasserstoffsäure, erforderlichenfalls in Gegenwart eines Kondensationsmittels, ausgehend von Anhydriden z.B. eines basischen Kondensationsmittels, wie eines Alkalimetallhydroxides oder -carbonats oder einer tertiären Stickstoffbase, z.B. eines Triniederalkylamins oder von Pyridin, und ausgehend von einer Säure, z.B. in Gegenwart einer Säure, wie einer Protonensäure, z.B. Chlorwasserstoff-, Schwefel-, Phosphor- oder einer Sulfonsäure, oder einer Lewissäure, z.B. Bortrifluorid-Etherat.

Ferner kann man freies Carboxy als Bestandteil von $R_3$ in üblicher Weise z.B. durch Behandeln mit einem Diazoniederalkan oder Triniederalkyloxonium-, Triniederalkylcarboxonium- oder Diniederalkylcarboniumsalz, wie Hexachloroantimonat oder Hexafluorophosphat, oder vor allem durch Umsetzung mit dem entsprechenden Alkohol oder einem reaktionsfähigen Derivat, wie einem Carbon-, Phosphorig-, Schweflig- oder Kohlensäureester, z.B. einem Niederalkancarbonsäureester, Triniederalkylphosphit, Diniederalkylsulfit oder dem Pyrocarbonat, oder einem Mineralsäure- oder Sulfonsäureester, z.B. dem Chlor- oder Bromwasserstoffsäure- oder Schwefelsäure-, Benzolsulfonsäure-, Toluolsulfonsäure- oder Methansulfonsäureester, des entsprechenden Alkohols oder einem davon abgeleiteten Olefin, zu einer veresterten Carboxylgruppe verestern.

Die Umsetzung mit dem entsprechenden Alkohol selbst kann vorteilhaft in Gegenwart eines sauren Katalysators erfolgen, wie einer Protonensäure, z.B. von Chlor- oder Bromwasserstoff-, Schwefel-, Phosphor-, Bor-, Benzolsulfon- und/oder Toluolsulfonsäure, oder eine Lewissäure,

z.B. von Bortrifluorid-Aetherat, in einem inerten Lösungsmittel, insbesondere einem Ueberschuss des eingesetzten Alkohols und erforderlichenfalls in Gegenwart eines wasserbindenden Mittels und/oder unter destillativer, z.B. azeotroper, Entfernung des Reaktionswassers und/oder bei erhöhter Temperatur.
Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Ueberschuss des eingesetzten Alkoholderivates oder des entsprechenden Alkohols. Ausgehend von einem Mineralsäure- oder Sulfonsäureester setzt man die zu veresternde Säure vorteilhaft in Form eines Salzes, z.B. des Natrium- oder Kaliumsalzes, und arbeitet erforderlichenfalls in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triäthylamin oder Pyridin, und/oder in einem inerten Lösungsmittel, wie einer der vorstehenden tertiären Stickstoffbasen oder eines polaren Lösungsmittels, z.B. in Dimethylformamid und/oder bei erhöhter Temperatur.

Die Umsetzung mit einem Olefin kann beispielsweise in Gegenwart eines sauren Katalysators, z.B. einer Lewissäure, z.B. von Bortrifluorid, einer Sulfonsäure, z.B. von p-Toluolsulfonsäure, oder vor allem eines basichen Katalysators, z.B. von Natrium oder Kaliumhydroxid, vorteilhaft in einem inerten Lösungsmittel, wie einem Aether, z.B. in Diäthyläther oder Tetrahydrofuran, erfolgen.

Eine freie Carboxylgruppe kann ferner durch Umsetzung mit Ammoniak oder einem mindestens ein Wasserstoffatom aufweisenden Amin in üblicher Weise, unter Dehydratisierung des intermediär gebildeten Ammoniumsalzes, z.B. durch azeotrope Destillation mit Benzol oder Toluol oder trockenes Erhitzen, in eine amidierte Carboxylgruppe überführt werden.

Die vorstehend beschriebenen Umwandlungen freier in veresterte oder amidierte Carboxylgruppen können aber auch so durchgeführt werden, dass man eine Verbindung der Formel I, worin $R_3$ Carboxy enthält, zunächst in üblicher Weise in ein reaktionsfähiges Derivat, beispielsweise mittels eines Halogenides des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol oder Amin in einen reaktiven Ester, d.h. Ester mit elektronenanziehenden Strukturen, wie den Ester mit Phenol, Thiophenol, p-Nitrophenol oder Cyanmethylalkohol, oder ein reaktives Amid, z.B. das von Imidazol oder 3,5-Dimethylpyrazol abgeleitete Amid, überführt und das erhaltene reaktionsfähige Derivat dann in üblicher Weise, z.B. wie nachstehend für die Umesterung bzw. gegenseitige Umwandlung veresterter und amidierter Carboxylgruppen beschrieben, mit einem entsprechenden Alkohol, Ammoniak oder dem entsprechenden mindestens ein Wasserstoffatom aufweisenden Amin zu der gewünschten Gruppe umsetzt.

Eine veresterte Carboxylgruppe kann in üblicher Weise, z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, zur freien Carboxylgruppe oder z.B. durch Umsetzung mit Ammoniak oder dem entsprechenden, mindestens ein Wasserstoffatom aufweisenden Amin in eine amidierte Carboxylgruppe überführt werden.

Eine veresterte Carboxylgruppe kann ferner in üblicher Weise, z.B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z.B.

von Natium- oder Kaliumhydroxid, doer einer starken Säure, wie einer Mineralsäure, z.B. von Salzsäure, Schwefelsäure, oder Phosphorsäure, oder einer organischen Sulfonsäure, z.B. von p-Toluolsulfonsäure, oder einer Lewissäure, z.B. von Brotrifluorid-Aetherat, zu einer anderen veresterten Carboxylgruppe umgeestert werden.

Eine amidierte Carboxylgruppe kann in üblicher Weise, z.B. durch Hydrolyse in Gegenwart eines Katalysators, beispielsweise einer starken Base, wie eines Alkalimetall- oder Erdalkalimetallhydroxides oder -carbonates, z.B. von Natrium- oder Kaliumhydroxid oder -carbonat, oder einer starken Säure, wie einer Mineralsäure, z.B. von Salzsäure, Schwefelsäure oder Phosphorsäure in die freie Carboxylgruppe umgewandelt werden.

Cyano kann hydrolytisch, z.B. unter basischen Bedingungen, zu Carbamyl oder Carboxy hydrolysiert oder durch Umsetzung mit einem Alkohol oder mit Ammoniak oder einem primären oder sekundären Amin in Gegenwart einer Säure, wie Chlorwasserstoffsäure und anschliessende Hydrolyse in verestertes bzw. amidiertes Carboxy überführt werden. Umgekehrt kann man Carbamyl durch Erhitzen, erforderlichenfalls in Gegenwart eines wasserbindenden Mittels, z.B. von Phosphorpentoxid oder von Polyphosphorsäure, oder eines N,N-disubstituierten Carbodiimides, z.B. von N,N-Dicyclohexycarbodiimid, zu Cyano dehydratisiert werden.

Weiterhin kann man gegebenenfalls in einer Ester-, Anhydrid- oder Salzform vorliegende Carboxyniederalkylreste der Formel $-C_mH_{2m}-COOH$ zu den entsprechenden Oxoniederalkylreste der Formel $-C_{m+1}H_{2m+1}=O$ bzw. Hydroxyniederalkylreste der Formel $-C_{m+1}H_{2m+2}-OH$ reduzieren, wobei m jeweils eine Zahl von 1 bis und mit 7, z.B. von 1 bis und mit 4, bedeutet.

Die Reduktion erfolgt in üblicher Weise, beispielsweise durch Umsetzung mit einem Hydridüberträger, der auf das α-ständige C-Atom Wasserstoff in anionischer Form überträgt, beispielsweise mit einem Leichtmetall- oder Dileichtmetallhydrides, z.B. mit Borhydrid-Aetherat oder vorzugsweise Lithiumaluminiumhydrid, vorteilhaft in einem Aether, wie einem Diniederalkyl- oder Niederalkylenäther, z.B. in Diäthyläther, Tertiärbutoxymethan oder Tetrahydrofuran, oder mittels Natriumborhydrid oder Natriumcyanoborhydrid, vorteilhaft in alkoholischer Lösung. Ebenso anwendbar sind Reduktionsmittel die nullwertige Metalle oder atomaren Wasserstoff übertragen. Dieser Prinzipien bedient man sich beispielsweise bei der metallischen Reduktion, die durch Einwirkung feinverteilter Metalle, z.B. von Zinkpulver, bewirkt werden kann, gleichermassen bei der Reduktion mit nascierendem Wasserstoff, der z.B. durch Säureeinwirkung auf unedle Metalle, wie von Essigsäure auf Zink, Eisen und Salzsäure oder Wassereinwirkung auf Natriumamalgam erzeugt werden kann. Durch die genannten Reduktionsmittel erhält man vorzugweise Verbindungen der Formel I, worin $R_3$ einen Hydroxyniederalkylrest bedeutet. Salzformen von Carboxy sind dabei beispielsweise Basesalzformen, wie Alkali- oder Erdalkalimetallsalzformen, können aber auch Ammoniumsalzformen mit Ammoniak oder organischen Aminen sein. Anhydridformen sind beispielsweise gemischte Anhydridformen mit Halogenwasserstoffsäuren, können aber auch gemischte Anhydridformen mit organischen Carbonsäuren, wie Niederalkansäuren sein. Esterformen sind beispielsweise Niederalkylesterformen, können aber auch andere organische Esterformen, wie gegebenenfalls substituierte Phenylesterformen sein. Als weitere Esterformen kommen Lactonformen, z.B. γ-Lactonformen, in Betracht, bei deren Vorliegen in $\omega$-Stellung und in einer niederen als der ($\omega$-2)-Stellung zwei Hydroxygruppen aufweisende Reste $R_3$ gebildet werden. Die Reduktion kann aber auch auf der Oxostufe aufgehalten werden, indem man selektive Reduktionsmittel verwendet. Solche sind für die Reduktion von gegebenenfalls in Salzform vorliegendem Carboxy beispielsweise

Mono- oder Diniederalkylborhydride, z.B. 2-(2,3-Dimethyl-butyl)-borhydrid, für die Reduktion von Anhydridformen beispielsweise Wasserstoff in Gegenwart von Palladium oder Cyanwasserstoffsäure in Gegenwart von tertiären aromatischen Stickstoffbasen, wie Chinolin, und für die Reduktion von Esterformen beispielsweise elektronegativ substituierte Aluminium- oder Alkalimetallaluminiumhydride, z.B. N-Methylpiperazinoaluminiumhydrid oder Natrium-bis-(2-methoxyäthoxy)-aluminiumhydrid, aber auch Dibutylaluminiumhydrid.

Umgekehrt kann man in Verbindungen der Formel I Gruppen $R_3$ der Formel $-C_{m+1}H_{2m+2}-OH$ zu Gruppen $R_3$ der Formel $-C_{m+1}H_{2m+}=O$ und Gruppen $R_3$ der Formeln $-C_mH_{2m}-CH_2OH$ bzw. $-C_mH_{2m}-CH=O$ zu solchen der Formel $C_mH_{2m}-COOH$ oxidieren, wobei m jeweils eine ganze Zahl von 1 bis und mit 7, z.B. von 1 bis und mit 4, bedruckt.

Die Oxidation von Gruppen der Formel $-C_{m+1}H_{2m+2}-OH$ zu solchen der Formel $-C_{m+1}H_{2m+2}=O$ erfolgt beispielsweise durch Behandeln mit einem für die Oxidation von primären und sekundären Alkoholen zu Aldehyden bzw. Ketonen üblichen Oxidationsmittel, wie nicht-oxidierenen Metallverbindungen, z.B. Chromylchlorid in Pyridin, Chromtrioxid in 2,5-Dimethylpyrazol, Pyrilinensalzen, Nitrosoniumtetrafluorborat oder den Kernplexen von Chlor bzw. Methansulfonsäureanhydrid und Dimethylsulfoxid, und weiteren, vor allem aber durch Behandeln mit einem aliphatischen oder cycloaliphatischen Keton, wie einem Oxoniederalkan, z.B. Aceton, oder einem Cycloalkanon, z.B. Cyclohexanon, in Gegenwart eines Aluminiumalkoholates, z.B. von Aluminiumisopropanolat.

Die Oxidation von Gruppen der Formeln $-C_mH_{2m}-CH_2OH$ bzw. $-C_mH_{2m}-CH=O$ zu solchen der Formel $-C_mH_{2m}-COOH$ erfolgt beispielsweise durch Behandeln mit einer für die Oxidation von Alkoholen und Aldehyden üblichen Oxidationsmittel, wie oxidierenden Schwermetallverbindungen, z.B. Mangan-IV- und Mangan-VII-, Chrom-VI-, Blei-IV- oder Wismut-III-Verbindungen, insbesondere mit Kaliumpermanganat, Chromtrioxid oder Chromsäure bzw. Alkalimetallchromaten. Weiterhin geeignet ist

Peroxyschwefelsäure, z.B. Caro'sche Säure, wobei bei Durchführung der Oxidation in einem Alkohol durch Veresterung der primär gebildeten Säure der entsprechende Ester erhalten wird.

Ferner kann man in Verbindungen der Formel I Reste der Formel $-S(O)_n-$, worin n für 0 steht, zu den entsprechenden Sulfinyl- oder Sulfonylresten, worin n für 1 oder 2 steht, Reste $-S(O)_n-$, worin n für 1 steht, zu den entsprechenden Sulfonylresten, worin n 2 bedeutet, und/oder Heteroarylreste $R_1$ und/oder $R_2$ - mit mindestens einem freien Ringstickstoffatom, wie Pyridylreste, N-oxidieren. Die Oxidation erfolgt vorzugsweise durch Einwirkung eines geeigneten Oxidationsmittels, vorteilhaft in einem diesem gegenüber inerten Lösungsmittel, erforderlichenfalls unter Kühlen oder Erwärmen, z.B. im Temperaturbereich von etwa -30° bis +100°C, vorzugsweise bei etwa 0° bis 60°C, in einem geschlossenen Gefäss und/oder unter Intergas, wie Stickstoff. Geeignete Oxidationsmittel sind beispielsweise Peroxyverbindungen, wie Wasserstoffperoxid, organische Hydroperoxide, z.B. tert.-Butylhydroperoxid, organische Persäuren, wie aromatische oder aliphatische Percarbonsäuren, z.B. m-Chlorperoxybenzoesäure, Peroxyessigsäure oder Permonophthalsäure, oxidierende Schwermetallverbindungen, wie Chrom-VI- oder Mangan-IV bzw. Mangan-VII-verbindungen, z.B. Chromtrioxid, Chromsäure, Mangandioxid oder Kaliumpermanganat, oxidierende anorganische Sauerstoffsäuren, wie Sauerstoffsäuren des Stickstoffs, der Halogene oder Chalkogene, oder deren Anhydride oder Salze, z.B. Salpetersäure, Distickstofftetroxid, Selendioxid oder Natriummetaperjodat, ferner Ozon. Geeignete Lösungsmittel sind beispielsweise halogenierte Kohlenwasserstoffe, wie Halogenalkane, z.B. Kohlenstofftetrachorid, Chloroform oder Methylenchlorid, oder Carbonsäuren, wie Alkansäuren, z.B. Essigsäure, oder deren Anhydride.

In einer bevorzugten Ausführungsform dieses Oxidationsverfahrens kann man beispielsweise Thioäther der Formel I, worin n 0 ist, und/oder ein Rest $R_1$, $R_2$ und/oder ein heteroarylaliphatischer Rest $R_3$ ein unsubstituiertes Ringstickstoffatom aufweist, durch Umsetzung mit einer organischen Persäure, z.B. mit m-Chlorperbenzoesäure, in einem Halogenalkan, z.B. in Chloroform, zu den entsprechenden Sulfinyl- bzw. in Tetrahydrofuran zu den entsprechenden Sulfonylverbindungen, worin n 1 bzw. 2 ist, oxidieren.

In einer anderen bevorzugten Ausführungsform kann man Thioäther der Formel I, worin n 0 ist, durch Behandeln mit Natriummetaperjodat, vorzugsweise in einem Halogenalkan, z.B. in Kohlenstofftetrachlorid oder Chloroform, selektiv zu den entsprechenden Sulfoxiden, in denen n und/oder m für 1 steht, oder diese mit Wasserstoffperoxid in Essigsäure zu Sulfonen, worin n 2 ist, und gewünschtenfalls Stickstoffatom oxidieren.

Umgekehrt kann man in Verbindungen der Formel I, worin n 1 oder 2 ist und/oder Heteroarylreste $R_1$ und/oder $R_2$ N-oxidiert sind, die Sulfinyl- bzw. Sulfonylgruppe zu reduzieren und/oder die Oxygruppe am N-oxidierten Ringstickstoff reduktiv entfernen. Die Reduktion erfolgt durch Behandeln mit üblichen Reduktionsmitteln, z.B. mit nascierendem oder katalytisch aktiviertem Wasserstoff, wie Eisen oder Zink und Säure, wie Salzsäure, oder mit Wasserstoff in Gegenwart von Raney-Nickel, vorteilhaft in einem inerten Lösungsmittel, wie einem Niederalkanol oder mit Leichtmetallhydriden bzw. Dileichtmetallhydriden, z.B. mit Alkalimetallaluminium- oder Alkalimetallborhydriden, z.B. mit Natriumborhydrid oder Lithiumaluminiumhydrid, vorteilhaft in einem inerten Lösungsmittel, wie einem Aether, z.B.

Diäthyläther oder Tetrahydrofuran, oder zur selektiven Reduktion von Gruppen N-Oxiden mit einer Phosphor-III-verbindung, wie einem Phosphin, z.B. Triphenylphosphin oder Tri-n-butylphosphin, oder einem Phosphorigsäureester, wie einem Triniederalkylphosphit, z.B. mit Trimethyl- oder Triäthylphosphit.

Ferner kann man in die Reste $R_1$ und/oder $R_2$ gegebenenfalls zusätzliche C-Substituenten einführen. So kann man in üblicher Weise halogenieren, z.B. durch Umsetzung mit Chlor oder Brom in Gegenwart von Eisen oder mittels N-Chlorsuccinimid. Ferner kann man in üblicher Weise, z.B. durch Umsetzung mit einem Alkylhalogenid, Alkanol oder Alken in Gegenwart von Aluminiumtrichlorid, alkylieren. Weiterhin kann man in üblicher Weise, z.B. mittels Salpetersäure/Schwefelsäure, nitrieren, die Nitrogruppe, z.B. mit Zinn-II-chlorid zu Amino reduzieren und diese mittels Natriumnitrit und Tetrafluorborsäure in Fluor, mittels Salzsäure, Natriumnitrit und Kupfer-I-chlorid (-bromid) durch Chlor (Brom), bzw. mittels Natriumnitrit und Kaliumjodid durch Jod ersetzen oder mittels Natriumnitrit und eines Niederalkylmercaptans in Niederalkylthio bzw. mittels Natriumnitrit in Hydroxy überführen.

In den Resten $R_3$ kann man ferner geminal gebundenes Diniederalkoxy, Diniederalkylthio, Niederalkylendioxy und Niederalkylendithio zu Oxo hydrolysieren, vorzugsweise säurekatalytisch.

Ferner kann man verestertes Hydroxy, wie Niederalkanoyloxy oder gegebenenfalls substituiertes Benzoyloxy aufweisende Reste $R_3$ zu den entsprechenden Hydroxy aufweisenden Resten hydrolysieren, z.B. wie vorstehend für die Hydrolyse von Verbindungen der Formel IXL angegeben.

Werden in erfindungsgemässen Verfahren Verbindungen der Formel I enthaltende Isomerengemische erhalten, können diese, ebenso wie erfindungsgemäss erhältliche Stereoisomerengemische, in üblicher Weise in die Komponenten aufgetrennt werden.

So können derartige Isomerengemische, z.B. Gemische erfindungsgemässer Stereoisomeren und Diastereomeren auf Grund der Unterschiede der physikalischen Eigenschaften der Komponenten durch übliche physikalische Trennverfahren, wie Kristallisations-, Chromatographie-, Destillations- oder Phasenverteilungsverfahren, in die Komponenten auftrennen.

Enantiomerengemische, wie Racemate, können durch Kristallisation aus optisch aktiven Lösungsmitteln, Chromatographie an optisch aktiven Feststoffen, Einwirkung von Mikroorganismen oder Umsetzung mit einer optisch aktiven Hilfsverbindung in Diastereomerengemische, z.B. mit einer optisch aktiven Säure in Gemische diastereomerer Säureadditionssalze, und Trennung derselben in die Diastereomeren aus denen die Enantiomeren in der jeweils üblichen Weise freigesetzt werden können in die Enantiomeren gespalten werden. Für diesen Zweck übliche optisch aktive Säuren sind z.B. D- oder L-Weinsäure, Di-o-Toluylweinsäure, Aepfelsäure,Mandelsäure, Camphersulfonsäuren oder Chinasäure.

Ferner können erhaltene freie Verbindungen in an sich bekannter Weise in Säureadditionssalze überführt werden, z.B. durch Umsetzen einer Lösung der freien Verbindung in einem geeigneten Lösungsmittel oder Lösungsmittelgemisch mit einer der vorgenannten Säuren oder mit einer Lösung davon, oder mit einem geeigneten Anionenaustauscher.

Erhaltene Salze können in an sich bekannter Weise in die freien Verbindungen umgewandelt werden, z.B. durch Behandeln eines basischen

Endstoffes mit einer Säure bzw. eines sauren Endstoffes mit einer Base, wie einem Alkalimetallhydroxid, einem Metallcarbonat oder -hydrogencarbonat, oder Ammoniak, oder mit einem geeigneten Anionenaustauscher.

Erhaltene Salze können in an sich bekannter Weise in andere Salze überführt werden, z.B. durch Behandlung eines Salzes mit einem geeigneten Metallsalz, wie einem Natrium-, Barium- oder Silbersalz, in einem geeigneten Lösungsmittel, in welchem ein sich bildendes anorganisches Salz unlöslich ist und damit aus dem Reaktionsgemisch ausscheidet, in andere Saäureadditionssalze übergeführt werden.

Die Verbindungen, einschliesslich ihrer Salze können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukte erhältlichen Verbindungen ausgeht und die fehlenden Verfahrensschritte ausführt, oder wobei ein Ausgangsstoff unter den Reaktionsbedingungen gebildet oder in Form eines Derivates davon, gegebenenfalls eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe sowie Verfahren zu ihrer Herstellung bilden ebenfalls einen Gegenstand der vorliegenden Erfindung.

Bei den erfindungsgemässen pharmazeutischen Präparaten, welche Verbindungen der Formel I oder pharmazeutisch verwendbare Salze davon enthalten, handelt es sich um solche zur enteralen wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Im Normalfall ist für einen etwa 75 kg schweren Warmblüter bei oraler Applikation eine ungefähre Tagesdosis von etwa 50-500 mg, vorteilhaft in mehreren gleichen Teildosen verteilt, zu veranschlagen.

Die neuen pharmazeutischen Präparate enthalten z.B. von etwa 10 % bis etwa 80 %, vorzugsweise von etwa 20 % bis etwa 60 % des Wirkstoffs,Erfindungsgemäss pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyopilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister

unter Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Starisäure oder Salze davon, wie Magnesium- oder Calciumstearat, und/oder Polyäthylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lachlösungen in geeigneten organischen Lösungsmitteln oder Lösungsmittelgemischen oder, zur Herstellung von Magensaftresistenten Ueberzügen, Lösungen von geeigneten Cellulosepräparaten, wie Acetylcellulosephthalat oder Hydroxypropylmethylcellulosephthalat, verwendet.

Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglycolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoff in Frage.

Zur parenteralen Verabreichung eigenen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von etwa 0,5 bis etwa 20 % des Wirkstoffs enthalten.

Creme sind Oel-in-Wasser-Emulsionen, die mehr als 50 % Wasser aufweisen. Ais ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl.Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukten davon, wie Polyglycerinfettsäureester oder Polyoxyäthylenfettalkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise

in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyäthylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.b. Vaseline, Paraffinöl und/oder Hartparafine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cereme und Salben mit sekretabsorbierenden Puderbestandteilen, wie Metalloxiden, z.B. Titanoxid oder Zinkoxid, ferner Talk und/oder Aluminiumsilikate, wleche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluoräthan, als Treibmittel

verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend liphophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glyckole, und/oder Polyäthylenglykol, als Feuchthaltemittel zur Herabsetzung der Verdunstung, und rückfettende Substanzen, wie Fettsäureester mit niedrigen Polyäthylenglycolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die vorliegende Erfindung betrifft ebenfalls die Verwendung der Verbindungen der Formeln I und der Salze von solchen Verbindungen mit salzbildenden Eigenschaften, vorzugsweise zur Behandlung von Entzündungen, in erster Linie von entzündlichen chronischen Erkrankungen des rheumatischen Formenkreises, besonders der chronischen Arthritis.

Die nachfolgenden Beispiele illustrieren die oben beschriebene Erfindung; sie sollen jedoch diese in ihrem Umfang in keiner Weise einschränken. Temperaturen sind in Celsiusgraden, Drucke in mbar angegeben.

Beispiel 1: 4,4 g (0,014 Mol) 1,5-Bis(p-methoxyphenyl)-3-mercapto-1H-1,2,4-triazol werden in 8 ml 2n- Natronlauge gelöst. Nach Zufügen von 2,5 ml Bromacetaldehyddimethylacetal in 30 ml Methanol wird 6 Stunden unter Rückfluss auf 70° erhitzt. Die Reaktionslösung wird mit 150 ml Wasser verdünnt und dreimal mit Methylenchlorid extrahiert. Die vereinigten Extrakte werden mit Wasser gewaschen, über Natriumsulfat getrocknet, filtriert und eingedampft. Der Rückstand liefert nach Kristallisation aus Methanol das 1,5-Bis(p-methoxyphenyl)-3-(2,2-dimethoxyäthylthio)-1H-1,2,4-triazol vom Smp. 107-108°.

Das Ausgangsmaterial kann z.B. folgendermassen hergestellt werden:

5,8 g p-Methoxyphenylhydrazin-hydrochlorid und 2,6 g Ammoniumrhodanid werden mit 20 ml Aethanol 20 Stunden unter Rühren und Rückfluss zum Sieden erhitzt. Nach Abkühlen, Nutschen, Waschen mit Wasser und Aethanol und anschliessendem Trocknen erhält man das p-Methoxyphenylthiosemicarbazid, Smp. 194-196°.

6,1 g p-Methoxyphenylthiosemicarbazid werden in 40 ml Pyridin suspendiert und bei -10° 5,8 g p-Methoxybenzoylchlorid so zugetropft, dass die Innentemperatur nicht über -5° steigt. Dann wird 20 Stunden bei Raumtemperatur gerührt und das Reaktionsgemisch auf 300 ml eiskalte 2n- Chlorwasserstoffsäure gegossen. Der erhaltene Niederschlag wird abgenutscht, mit Wasser und Methanol gewaschen und getrocknet. Man erhält $N^1,N^3$-Bis(p-methoxyphenyl)-thiosemicarbazid vom Smp. 154-159°.

7,5 g $N^1$-(4-Methoxyphenyl)-$N^3$-benzoyl-thiosemicarbazid werden mit 75 ml 2-n. Natronlauge während 3 Stunden unter Rückfluss zum Sieden erhitzt. Die erhaltene klare Lösung wird abgekühlt und mit 75 ml 2-n. Chlorwasserstoffsäure versetzt. Man erhält einen kristallinen Niederschlag, der abgenutscht, mit Wasser und Aethanol gewaschen und getrocknet wird. Das so hergestellte 1,5-Bis(p-methoxyphenyl)-3-mercapto-1H-1,2,4-triazol schmilzt bei 173-174°.

Beispiel 2: In analoger Weise wie in Beispiel 1 beschrieben erhält man ausgehend von 2,2 g (0,007 Mol) 1,5-Bis(p-methoxyphenyl)-3-mercapto-1H-1,2,4-triazol, und der entsprechenden Menge Natronlauge sowie 1,53 g Bromessigsäuremethylester das 1,5-Bis(p-methoxyphenyl)-3-methoxycarbonylmethylthio-1H-1,2,4-triazol vom Smp. 78-79° (aus Petroläther).

Beispiel 3: In analoger Weise wie in Beispiel 1 beschrieben erhält man ausgehend von 1,5 g (0,005 Mol) 1,5-Bis(p-methoxyphenyl)-3-mercapto-1H-1,2,4-triazol und der entsprechenden Menge Natronlauge sowie 0,75 g 3-Brompropionsäure, nach Ansäuern der Reaktionslösung mit verdünnter Chlorwasserstoffsäure, das 1,5-Bis(p-methoxyphenyl)-3-(2-carboxyäthylthio)-1H-1,2,4-triazol vom Smp. 85-86° (aus Aethanol/Wasser).

Beispiel 4: In analoger Weise wie in Beispiel 1 beschrieben erhält man ausgehend von 3,1 g (0,01 Mol) 1,5-Bis(p-methoxyphenyl)]-3-mercapto-1H-1,2,4-triazol und der entsprechenden Menge Natronlauge sowie 1,8 g 3-Brompropionaldehydäthylenacetal das 1,5-Bis(p-methoxyphenyl)-3-(3,3-äthylendioxypropylthio)-1H-1,2,4-triazol vom Smp. 94-96° (aus Aether).

Beispiel 5: 2,5 g (0,0062 Mol) 1,5-Bis(p-methoxyphenyl)-3-(2,2-dimethoxyäthylthio)-1H-1,2,4-triazol werden in einem Gemisch von 20 ml Essigsäure, 10 ml Wasser und 5 ml Schwefelsäure gelöst und 20 Stunden bei 20° aufbewahrt. Man verdünnt mit 200 ml Wasser und extrahiert zweimal mit Essigsäureäthylester. Die vereinigten Extrakte werden mehrmals mit Wasser gewaschen, über Natriumsulfat getrocknet, filtriert und eingedampft. Nach Kristallisation aus Aether erhält man das 1,5-Bis(p-methoxyphenyl)-3-(2-oxoäthylthio)-1H-1,2,4-triazol vom Smp. 88-89°.

Beispiel 6: 2,5 g (0,0065 Mol) 1,5-Bis(p-methoxyphenyl)-3-(methoxycarbonylmethylthio)-1H-1,2,4-triazol werden mit 7 ml 1n- Natronlauge 1 1/2 Stunden unter Rühren und Rückfluss erhitzt. Die abgekühlte Lösung wird mit 7 ml 2n- Chlorwasserstoffsäure versetzt. Die abgeschiedenen Kristalle werden filtriert, mit Wasser gewaschen und getrocknet. Das erhaltene 1,5-Bis(p-methoxyphenyl)-3-carboxymethylthio-1H-1,2,4-triazol schmilzt bei 184-185°.

Beispiel 7: In analoger Weise wie in Beispiel 5 beschrieben erhält man aus 1,8 g 1,5-Bis(p-methoxyphenyl)-3-(3,3-äthylendioxypropylthio)-1H-1,2,4-triazol nach Hydrolyse mit einem Gemisch von 20 ml Essigsäure, 10 ml Wasser und 5 ml Schwefelsäure nach dem Aufarbeiten das 1,5-Bis(p-methoxyphenyl)-3-(3-oxopropylthio)-1H-1,2,4-triazol vom Smp. 103-105° (aus Aether).

Beispiel 8: 1,1 g (0,003 Mol) 1,5-Bis(p-methoxyphenyl)-3-carboxymethylthio-1H-1,2,4-triazol und 0,5 ml Thionylchlorid in 30 ml Toluol werden unter Rühren auf 60-70° erhitzt bis eine klare Lösung entsteht. Dann wird das Lösungsmittel unter vermindertem Druck abdestilliert und der Rückstand wieder in Toluol gelöst. Diese Lösung wird zu einer auf 0° gekühlten, gerührten Lösung von 1,2 g Dimethylamin in 50 ml Toluol getropft. Anschliessend lässt man die Temperatur des Lösungsmittels auf Raumtemperatur steigen. Dann wird unter vermindertem Druck eingedampft und der Rückstand

zwischen Aethylacetat und Wasser verteilt. Nach mehrmaligem Waschen mit Wasser wird die organische Phase mit Natriumsulfat getrocknet, filtriert und eingedampft. Nach Kristallisation des Rückstands aus Aethanol erhält man das 1,5-Bis(p-methoxyphenyl)-3-N,N-dimethylcarbamoylmethylthio-1H-1,2,4-triazol vom Smp. 173-175°. (Ausbeute 30 %).

Analog, jedoch unter Verwendung von Ammoniak statt Dimethylamin, erhält man das 1,5-Bis(p-methoxyphenyl)-3-carbamoylmethylthio-1H-1,2,4-triazol, Smp. 170-172°.

Beispiel 9: Eine Lösung von 3,0 g (0,0078 Mol) 1,5-Bis(p-methoxyphenyl)-3-methoxycarbonylmethylthio-1H-1,2,4-triazol in 50 ml Dichlormethan wird portionsweise mit 2,0 g 90%-iger m-Chlorperbenzoesäure versetzt. Nach 3 Tagen Rühren bei Raumtemperatur wird das Lösungsmittel unter vermindertem Druck abdestilliert. Der Rückstand wird aus 100 g Kieselgel 60 (230-400 mesh, Merck) unter Anwendung eines Druckes von 0,6 bar und Toluol-Aethylacetat (4:1, v/v) als Fliessmittel chromatographiert. Die das gewünschte Produkt enthaltenden Fraktionen werden vereinigt und eingedampft. Man erhält so das 1,5-Bis(p-methoxyphenyl)-3-methoxycarbonylmethansulfinyl-1H-1,2,4-triazol als farbloses Oel, 1H-NMR-Spektrum ($CDCl_3$, TMS): Singulett 2H bei $\delta$ = 4,37 ppm, welches sich in den zuletzt erhaltenen Fraktionen befindet. Aus den mittleren Fraktionen erhält man das 1,5-Bis(p-methoxyphenyl)-3-methoxycarbonylmethansulfonyl-1H-1,2,4-triazol 1H-NMR-Spektrum ($CDCl_3$, TMS): Singulett 2 H bei $\delta$ = 4,41 ppm als farbloses Oel.

Beispiel 10:

1,5 g (0,00375 Mol) 1,5-Bis(p-methoxyphenyl)-3-methoxycarbonylmethansulfinyl-1H-1,2,4-triazol werden mit 40 ml 1-n Natronlauge 20 Stunden lang gerührt. Die Lösung wird, nach Behandlung mit Tierkohle, mit 4,1 ml 1-n. Chlorwasserstoffsäure versetzt und anschliessend mit Methylenchlorid extrahiert. Die Extrakte werden mit Natriumsulfat getrocknet, filtriert und unter vermindertem Druck eingedampft. Das zurückbleibende, ölige 1,5-Bis(p-methoxyphenyl)-3-carboxymethansulfinyl-1H-1,2,4-triazol

wird in der berechneten Menge 1,0-n. Natronlauge gelöst und die Lösung lyophilisiert. Das Lyophilisat wird mit wenig Isopropanol angeschlämmt und abgenutscht. Man erhält so das Natriumsalz des 1,5-Bis(p-methoxyphenyl)-3-carboxymethansulfinyl-1H-1,2,4-triazols, als Dihydrat, welches ab 135° unter Zersetzung schmilzt.

In analoger Weise erhält man ausgehend von 1,5-Bis(p-methoxyphenyl)-3-methoxycarbonylmethansulfonyl-1H-1,2,4-triazol das Natriumsalz des 1,5-Bis(p-methoxyphenyl)-3-carboxymethansulfonyl-1H-1,2,4-triazols, welches ab 133° unter Zersetzung schmilzt.

Beispiel 11: 7,5 g (0,0226 Mol) $N^1$-(4-Methoxyphenyl)-$N^3$-benzoylthiosemicarbazid und 2,7 g Natriumchloracetat werden mit 75 ml 2-n. Natronlauge während 3 Stunden unter Rückfluss zum Sieden erhitzt. Die erhaltene klare Lösung wird abgekühlt und mit 75 ml 2-n. Chlorwasserstoffsäure versetzt. Man erhält einen kristallinen Niederschlag, der abgenutscht, mit Wasser und Aethanol gewaschen wird. Das so hergestellte 1,5-Bis(p-methoxyphenyl)-3-carboxymethylthio-1H-1,2,4-triazol schmilzt bei 184-185°.

Beispiel 12: 2,2 g (0,007 Mol) 1,5-Bis(p-methoxyphenyl)-3-mercapto-1H-1,2,4-triazol werden in 15 ml 2-n. Natronlauge gelöst. Nach zufügen von 1,3 g Brommalonsäure wird die erhaltene Lösung bei Raumtemperatur 24 Stunden stehen gelassen. Dann stellt man mit konzentrierter Chlorwasserstoffsäure auf pH 1 und erhitzt anschliessend 1 Stunde unter Rühren und Rückfluss zum Sieden. Nach Abkühlen und Stehenlassen filtriert man das abgeschiedene 1,5-Bis(p-methoxyphenyl)-3-carboxymethylthio-1H-1,2,4-triazol ab; Smp. 184-185°.

Beispiel 13: In analoger Weise wie in Beispiel 1 beschrieben erhält man aus 2,2 g (0,007 Mol) 1,5-Bis(p-methoxyphenyl)-3-mercapto-1H-1,2,4-triazol und der entsprechenden Menge Natronlauge sowie Chloracetonitril das 1,5-Bis(p-methoxyphenyl)-3-cyanomethylthio-1H-1,2,4-triazol als Oel.

Beispiel 14: In analoger Weise wie in den Beispielen 1 und 6 beschrieben erhält man durch Umsetzung von 1,5-Bis(p-methoxyphenyl)-3-mercapto-1H-1,2,4-triazol mit der berechneten Menge 2-Brompropionsäureäthylester das 1,5-Bis(p-methoxyphenyl)-3-(1-äthoxycarbonyläthylthio)-1H-1,2,4-triazol vom Smp. 80-82° und aus diesem das 1,5-Bis(p-methoxyphenyl)-3-(1-carboxyäthylthio)-1H-1,2,4-triazol vom Smp. 148-150°.

Beispiel 15: 1,76 g (0,005 Mol) 1,5-Bis(p-methoxyphenyl)-3-cyanomethylthio-1H-1,2,4-triazol werden mit 10 ml Essigsäure und 10 ml 36%-iger Chlorwasserstoffsäure 12 Stunden unter Rückfluss erhitzt. Dann wird das Reaktionsgemisch unter vermindertem Druck zur Trockne eingedampft. Der Rückstand wird aus Chloroform-Aethanol-Gemisch kristallisiert. Man erhält das 1,5-Bis(p-methoxyphenyl)-3-carboxymethylthio-1H-1,2,4-triazol vom Smp. 184-185°.

Beispiel 16: 1,76 g (0,05 Mol) 1,5-Bis(p-methoxyphenyl)-3-cyanomethylthio-1H-1,2,4-triazol werden mit 20 ml gesättigter methanolischer Chlorwasserstoffsäure unter Rühren im Druckrohr 10 Stunden auf 100° erhitzt. Dann wird zur Trockne eingedampft, den Rückstand mit Diäthyläther mehrmals ausgezogen, die Auszüge filtriert und teilweise eingedampft. Nach Zusatz von Petroläther kristallisiert das 1,5-Bis(p-methoxyphenyl)-3-methoxycarbonymethylthio-1H-1,2,4-triazol, Smp. 78-79°.

Beispiel 17: Zu einer Lösung von 0,45 g (0,005 Mol) Thioglykolsäure in 5 ml Dimethylformamid werden 0,54 g Natriummethylat eingetragen. Es wird bei Raumtemperatur 1 Stunde gerührt, dann werden 1,8 g (0,005 Mol) 1,5-Bis(p-methoxyphenyl)-3-methylsulfonyl-1H-1,2,4-triazol eingetragen. Anschliessend erhitzt man 24 Stunden unter Rühren auf 100°. Das Lösungsmittel wird unter vermindertem Druck abdestilliert und der Rückstand mit 5 ml 2-n. Chlorwasserstoffsäure versetzt. Man extrahiert mehrmals mit Dichlormethan, trocknet die Extrakte über Natriumsulfat, filtriert und dampft ein. Das zurückbleibende rohe 1,5-Bis-(p-methoxyphenyl)-3-carboxymethylthio-1H-1,2,4-triazol kristallisiert aus Chloroform-Aethanol mit dem Smp. 184-185°.

Beispiel 18: Zu 1,93 g (0,01 Mol) 4-Methoxybenzoylisothiocyanat (C.A. 44 2515 b) in 50 ml werden 1,10 g Mercaptoessigsäuremethylester getropft. Anschliessend rührt man 5 Stunden bei Raumtemperatur. Das Lösungsmittel wird unter vermindertem Druck abdestilliert. Dann werden 1,40 g 4-Methoxyphenylhydrazin und 100 ml Benzol zugefügt. Nach 4 Stunden Rühren und Rückflusssieden am Wasserabscheider wird das Lösungsmittel unter vermindertem Druck abdestilliert. Durch Kristllisation des Rückstands aus Diäthyläther unter Zusatz von Petroläther erhält man das 1,5-Bis(p-methoxyphenyl)-3-methoxycarbonylmethylthio-1H-1,2,4-triazol vomSmp. 78-79°.

Beispiel 19: In analoger Weise wie in den Beispielen 1 bis 18 beschrieben erhält man ferner:
1,5-Bis(p-chlorphenyl)-3-(2,2-dimethoxyäthylthio)-1H-1,2,4-triazol vom Smp. 117-118°,
1,5-Bis(p-chlorphenyl)-3-(2-oxoäthylthio)-1H-1,2,4-triazol vom Smp. 96-100°,
1-(3-Pyridyl)-5-phenyl-3-(2-hydroxyäthylthio)-1H-1,2,4-triazol als Oel, und
1,5-Bis(p-methoxyphenyl)-3-(2-hydroxyäthylthio)-1H-1,2,4-triazol vom Smp. 140-142°.

Beispiel 20: Tabletten, enthaltend 25 mg Wirkstoff, z.B. 3-(2,2-Dimethoxyäthylthio)-1,5-bis(p-methoxyphenyl)-1H-1,2,4-triazol, können folgendermassen hergestellt werden.

| Bestandteile (für 1000 Tabletten): | |
|---|---|
| Wirkstoff | 25,0 g |
| Lactose | 100,7 g |
| Weizstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 21: Kautabletten, enthaltend 30 mg Wirkstoff, z.B. 3-(2,2-Dimethoxyäthylthio)-1,5-bis(p-methoxyphenyl)-1H-1,2,4-triazol, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%-ige Gelatinelösung | q.s. |

Herstellung: Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50° getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das

Ganze gründlich vermischt und zu beidseitig konkaven Tabletten von etwa 10 mm Durchmesser mit Bruchrille auf der Oberseite verpresst.

Beispiel 22: Tabletten enthaltend 100 mg Wirkstoff, z.B. 3-(2,2-Dimethoxyäthylthio)-1,5-bis(p-methoxyphenyl)-1H-1,2,4-triazol können folgendermassen hergestellt werden:

Zusammensetzung (für 1000 Tabletten):

| | |
|---|---|
| Wirkstoff | 100,0 g |
| Lactose | 248,5 g |
| Maisstärke | 17,5 g |
| Polyäthylenglykol 6000 | 5,0 g |
| Talkum | 15,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 23: In analoger Weise wie in den Beispielen 20-22 können auch pharmazeutische Präparate enthaltend jeweils eine der in den Beispielen 2 bis 19 genannten Verbindungen der Formel I hergestellt werden, wobei Verbindungen, worin $R_3$ Carboxy ist, auch in Form von Salzen mit Basen, z.B. als Natriumsalz, vorliegen können.

Patentansprüche für die Vertragsstaaten: DE, GB, FR, CH, LI, IT, NL, BE, SE, LU

1. Neue 3-Mercapto-1,2,4-triazacycloalkadienderivate der Formel I

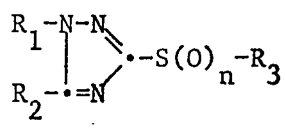

(I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/-oder Trifluormethyl substituierte Aryl- oder gegebenenfalls N-oxidierte Heteroarylreste bedeuten, n für 0, 1 oder 2 steht und $R_3$ einen durch eine gegebenenfalls veresterte oder amidierte Carboxygruppe, Cyano oder in höherer als der α-Stellung durch eine oder zwei gegebenenfalls veresterte oder verätherte Hydroxygruppe(n), eine oder zwei verätherte Mercaptogruppe(n), eine oxidierte Mercaptogruppe oder eine Oxogruppe substituierten aliphatischen Kohlenwasserstoffrest bedeutet, und ihre Salze.

2. Verbindungen gemäss Anspruch 1, der Formel I, worin die Reste $R_1$ und $R_2$ unabhängig voneinander unsubstituierte oder durch Niederalkyl, Niederalkoxy bzw. an vicinalen C-Atomen Niederalkyl(id)endioxy, Hydroxy, Halogen, Niederalkanoyloxy, Niederalkylthio, Niederalkansulfinyl, Niederalkansulfonyl, Amino, Mono- oder Diniederalkylamino und/oder Trifluormethyl substituierte 6 bis und mit 10 C-Atomen aufweisende Aryl- bzw. monocyclische, ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedrige Heteroarylreste bedeuten, n für 0, 1 oder 2 steht und $R_3$ Carboxyniederalkyl, Niederalkoxycarbonylniederalkyl, Carbamylniederalkyl, N-Mono- und N,N-Diniederalkylcarbamylniederalkyl,

Cyanoniederalkyl oder die Hydroxy-, Niederalkanoyloxy-, Niederalkoxy-, Niederalkylendioxy-, Niederalkylthio-, Niederalkylendithio, Niederalkansulfinyl-, Niederalkansulfonyl- bzw. Oxogruppe(n) in höherer als der α-Stellung tragendes Mono- oder Dihydroxyniederalkyl, Niederalkanoyloxyniederalkyl, Mono- oder Diniederalkoxyniederalkyl, Niederalkylendioxyniederalkyl, Niederalkylendithioniederalkyl, Mono- oder Diniederalkylthioniederalkyl, Niederalkansulfinylniederalkyl, Niederalkansulfonylniederalkyl oder Oxoniederalkyl bedeutet, und ihre Salze.

3. Verbindungen gemäss Anspruch 1, der Formel I, worin die Reste $R_1$ und $R_2$ unabhängig voneinander unsubstituiertes oder durch Niederalkyl, Niederalkoxy, Hydroxy, Halogen, Niederalkanoyloxy, Niederalkylthio, Niederalkansulfonyl, Diniederalkylamino und/oder Trifluormethyl substituiertes Phenyl bzw. unsubstituiertes oder durch Niederalkyl, Niederalkoxy, Halogen oder Hydroxy substituiertes Furyl, Thienyl, Pyridyl bzw. 1-Oxidopyridyl bedeuten, n für 0, 1 oder 2 steht und $R_3$ Carboxyniederalkyl, Niederalkoxycarbonylniederalkyl, Carbamylniederalkyl, N-Mono- oder N,N-Diniederalkylcarbamylniederalkyl oder die Hydroxy-, Niederalkoxy-, Niederalkylendioxy-, Niederalkylidendioxy- bzw. Oxogruppe(n) in höherer als der α-Stellung tragendes Hydroxyniederalkyl, Mono- oder Diniederalkoxyniederalkyl, Niederalkylendioxyniederalkyl, Niederalkylidendioxyniederalkyl oder Oxoniederalkyl bedeutet, und ihre Salze.

4. Verbindungen gemäss Anspruch 1, der Formel I, worin $R_1$ und $R_2$ durch Niederalkoxy mit bis und mit 4 C-Atomen substituierte Phenylreste bedeuten, n für 0, 1 oder 2 steht und $R_3$ die Carboxy oder Niederalkoxycarbonylgruppe in α-Stellung tragendes Carboxy- bzw. Niederalkoxycarbonylniederalkyl mit bis und mit 4 C-Atomen im Niederalkyl- und gegebenenfalls Niederalkoxyteil oder die Hydroxy-, Oxo-, Niederalkoxy- bzw. Niederalkylendioxygruppe in höherer als der α-Stellung tragendes Hydroxyniederalkyl mit 2 bis und mit 4 C-Atomen, Oxoniederalkyl mit 2 bis und mit 4 C-Atomen oder Mono- oder Diniederalkoxyniederalkyl bzw. Niederalkylendioxyniederalkyl mit jeweils bis und mit 4 C-Atomen in jedem Alkyl(en)teil bedeutet, und ihre Salze.

5. Verbindungen gemäss Anspruch 1, der Formel I, worin $R_1$ und $R_2$ durch Niederalkoxy mit bis und mit 4 C-Atomen substituierte Phenylreste bedeuten, n für 0, 1 oder 2 steht und $R_3$ die Carboxy oder Niederalkoxycarbonylgruppe in α-Stellung tragendes Carboxy- bzw. Niederalkoxycarbonylniederalkyl mit bis und mit 4 C-Atomen im Niederalkyl- und gegebenenfalls Niederalkoxyteil bedeutet, und ihre Salze.

6. 1,5-Bis(p-methoxyphenyl)-3-(2,2-dimethoxyäthylthio)-1H-1,2,4-triazol gemäss Anspruch 1 oder ein Salz davon.

7. 1,5-Bis(p-methoxyphenyl)-3-(2-oxoäthylthio)-1H-1,2,4-triazol gemäss Anspruch 1 oder ein Salz davon.

8. 1,5-Bis(p-methoxyphenyl)-3-carboxymethylthio-1H-1,2,4-triazol gemäss Anspruch 1 oder ein Salz davon.

9. 1,5-Bis(p-methoxyphenyl)-3-(3-oxopropylthio)-1H-1,2,4-triazol, 1,5-Bis(p-methoxyphenyl)-3-methoxycarbonylmethylthio-1H-1,2,4-triazol, 1,5-Bis(p-methoxyphenyl)-3-(2-carboxyäthylthio)-1H-1,2,4-triazol, 1,5-Bis(p-methoxyphenyl)-3-(3,3-äthylendioxypropylthio)-1H-1,2,4-triazol,1,5-Bis(p-chlorphenyl)-2-(2,2-dimethoxyäthylthio)-1H-1,2,4-triazol, 1,5-Bis(p-chlorphenyl)-3-(2-oxoäthylthio)-1H-1,2,4-triazol, 1-(3-Pyridyl)-5-phenyl-3-(2-hydroxyäthylthio)-1H-1,2,4-triazol gemäss Anspruch 1 oder jeweils ein Salz davon.

10. 1,5-Bis(p-methoxyphenyl)-3-methoxycarbonylmethansulfinyl-1H-1,2,4-triazol, 1,5-Bis(p-methoxyphenyl)-3-carboxymethansulfinyl-1H-1,2,4-triazol, 1,5-Bis(p-methoxyphenyl)-3-cyanomethylthio-1H-1,2,4-triazol, 1,5-Bis(p-methoxyphenyl)-3-N,N-dimethylcarbamoylmethylthio-1H-1,2,4-triazol, 1,5-Bis(p-methoxyphenyl)-3-methoxycarbonylmethansulfonyl-1H-1,2,4-triazol, 1,5-Bis(p-methoxyphenyl)-3-carboxymethansulfonyl-1H-1,2,4-triazol, 1,5-Bis(p-methoxyphenyl)-3-(1-äthoxycarbonyläthylthio)-1H-1,2,4-triazol, 1,5-Bis(p-methoxyphenyl)-3-(1-carboxyäthylthio)-1H-1,2,4-triazol, 1,5-Bis(p-methoxyphenyl)-3-carbamoylmethylthio-1H-1,2,4-triazol oder 1,5-Bis(p-methoxyphenyl)-3-(2-hydroxyäthylthio)-1H-1,2,4-triazol gemäss Anspruch 1 oder jeweils ein Salz davon.

11. Pharmazeutische Präparate, enthaltend eine Verbindung gemäss einem der Patentansprüche 1-10 neben üblichen pharmazeutischen Hilfsstoffen.

12. Verfahren zur Herstellung neuer 3-Mercapto-1,2,4-triazacycloalkadienderivate der Formel I

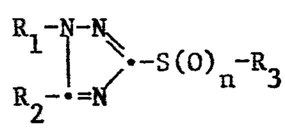

(I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/-oder Trifluormethyl substituierte Aryl- oder gegebenenfalls N-oxidierte Heteroarylreste bedeuten, n für 0, 1 oder 2 steht und $R_3$ einen durch eine gegebenenfalls veresterte oder amidierte Carboxygruppe, Cyano oder in höherer als der α-Stellung durch eine oder zwei gegebenenfalls veresterte oder verätherte Hydroxygruppe(n), eine oder zwei verätherte Mercaptogruppe(n), eine oxidierte Mercaptogruppe oder eine Oxogruppe substituierten aliphatischen Kohlenwasserstoffrest bedeutet, und ihrer Salze, dadurch gekennzeichnet, dass man

a) aus einer Verbindung der Formel

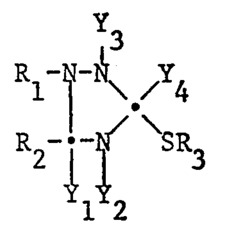

(II),

worin einer der Reste $Y_1$ und $Y_2$ einen abspaltbaren Rest Y und der andere ein Wasserstoffatom bedeutet und $Y_3$ und $Y_4$ gemeinsam eine zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzlichen Bindung HY abspaltet oder

b) Verbindungen der Formel

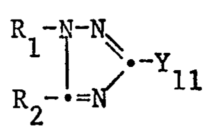

(XXXI) und $Y_{12} - R_3$ (XXXII),

worin $Y_{11}$ eine gegebenenfalls in Salzform vorliegende Mercaptogruppe und $Y_{12}$ reaktionsfähiges verestertes Hydroxy bedeutet oder $Y_{11}$ Sulfonyl und $Y_{12}$ eine gegebenenfalls in Salzform vorliegende Mercaptogruppe darstellt, miteinander zur entsprechenden Verbindung der Formel I, worin n für 0 steht, kondensiert oder

c) Verbindungen der Formeln

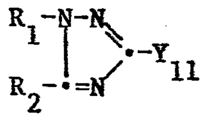 (XXXI) und $Y_{12}-R_3$ (XXXII),

worin einer der Reste $Y_{11}$ und $Y_{12}$ einen metallischen Rest und der andere eine Gruppe $S(O)_n-Y_{13}$ bedeutet, in der $Y_{13}$ Halogen darstellt, miteinander kondensiert oder

d) eine Verbindung der Formel

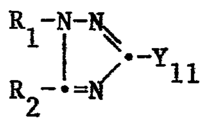 (XXXI),

worin $Y_{11}$ Mercapto ist, oder ein Salz davon mit einem von einer Verbindung der Formel $R_3$-H (XXXVII) abgeleiteten vicinalen Epoxiderivat zu einer Verbindung der Formel I umsetz, worin $R_3$ in β-Stellung eine Hydroxygruppe aufweist und n 0 ist, oder

e) eine Verbindung der Formel

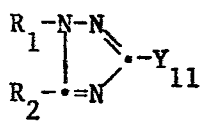 (XXXI),

worin $Y_{11}$ Mercapto ist, oder ein Salz davon mit einer Verbindung der Formel $R_3'$-H (XXXVIII), worin $R_3'$ ein von $R_3$ abgeleiteter, mindestens in α,β-Stellung eine C-C-Doppel- oder C-C-Dreifachbindung aufweisenden aliphatischen Rest bedeutet, zu einer Verbindung der Formel I umsetzt, worin $R_3$ α,β-gesättigt oder α,β-einfach ungesättigt ist und n 0 ist, oder

f) in einer Verbindung der Formel

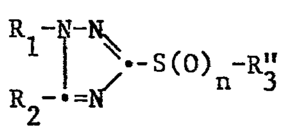 (IXL),

worin $R_3''$ einen in eine Gruppe $R_3$ überführbaren Rest bedeutet, $R_3''$ in die gewünschte Gruppe $R_3$ überführt, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

13. Die gemäss dem Verfahren des Anspruchs 12 erhältlichen Verbindungen.

14. Eine Verbindung gemäss einem der Ansprüche 1-10 zur Anwendung in einem Verfahren zur therapeutischen Behandlung des menschlichen oder tierischen Körpers.

15. Eine Verbindung gemäss einem der Ansprüche 1-10 als analgetisches Mittel.

FO 7.4 KVB/gb*

Patentansprüche für den Vertragsstaat: AT

1. Verfahren zur Herstellung neuer 3-Mercapto-1,2,4-triazacycloalkadienderivate der Formel I

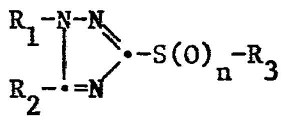

(I),

worin die Reste $R_1$ und $R_2$ unabhängig voneinander unsubstituierte oder durch aliphatische Kohlenwasserstoffreste, freies, veräthertes oder verestertes Hydroxy, gegebenenfalls S-oxidiertes veräthertes Mercapto, freies oder aliphatisch substituiertes Amino, Nitro und/-oder Trifluormethyl substituierte Aryl- oder gegebenenfalls N-oxidierte Heteroarylreste bedeuten, n für 0, 1 oder 2 steht und $R_3$ einen durch eine gegebenenfalls veresterte oder amidierte Carboxygruppe, Cyano oder in höherer als der α-Stellung durch eine oder zwei gegebenenfalls veresterte oder verätherte Hydroxygruppe(n), eine oder zwei verätherte Mercaptogruppe(n), eine oxidierte Mercaptogruppe oder eine Oxogruppe substituierten aliphatischen Kohlenwasserstoffrest bedeutet, und ihrer Salze, dadurch gekennzeichnet, dass man

a) aus einer Verbindung der Formel

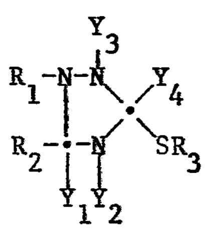

(II),

worin einer der Reste $Y_1$ und $Y_2$ einen abspaltbaren Rest Y und der andere ein Wasserstoffatom bedeutet und $Y_3$ und $Y_4$ gemeinsam eine

zusätzliche Bindung darstellen, oder worin $Y_4$ einen abspaltbaren Rest Y und $Y_3$ Wasserstoff bedeutet und $Y_1$ und $Y_2$ eine zusätzliche Bindung darstellen, oder aus einem Salz davon unter Einführung einer zusätzlichen Bindung HY abspaltet oder

b) Verbindungen der Formel

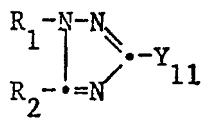 (XXXI) und $Y_{12} - R_3$ (XXXII),

worin $Y_{11}$ eine gegebenenfalls in Salzform vorliegende Mercaptogruppe und $Y_{12}$ reaktionsfähiges verestertes Hydroxy bedeutet oder $Y_{11}$ Sulfonyl und $Y_{12}$ eine gegebenenfalls in Salzform vorliegende Mercaptogruppe darstellt, miteinander zur entsprechenden Verbindung der Formel I, worin n für 0 steht, kondensiert oder

c) Verbindungen der Formeln

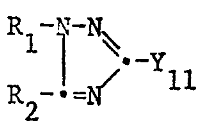 (XXXI) und $Y_{12}-R_3$ (XXXII),

worin einer der Reste $Y_{11}$ und $Y_{12}$ einen metallischen Rest und der andere eine Gruppe $S(O)_n-Y_{13}$ bedeutet, in der $Y_{13}$ Halogen darstellt, miteinander kondensiert oder

d) eine Verbindung der Formel

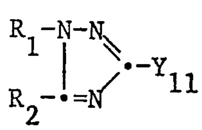 (XXXI),

worin $Y_{11}$ Mercapto ist, oder ein Salz davon mit einem von einer Verbindung der Formel $R_3$-H (XXXVII) abgeleiteten vicinalen Epoxiderivat zu einer Verbindung der Formel I umsetz, worin $R_3$ in β-Stellung eine Hydroxygruppe aufweist und n 0 ist, oder

e) eine Verbindung der Formel

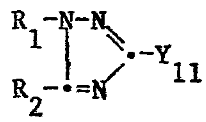

(XXXI),

worin $Y_{11}$ Mercapto ist, oder ein Salz davon mit einer Verbindung der Formel $R'_3$-H (XXXVIII), worin $R'_3$ ein von $R_3$ abgeleiteter, mindestens in α,β-Stellung eine C-C-Doppel- oder C-C-Dreifachbindung aufweisenden aliphatischen Rest bedeutet, zu einer Verbindung der Formel I umsetzt, worin $R_3$ α,β-gesättigt oder α,β-einfach ungesättigt ist und n 0 ist, oder

f) in einer Verbindung der Formel

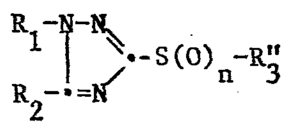

(IXL),

worin $R''_3$ einen in eine Gruppe $R_3$ überführbaren Rest bedeutet, $R''_3$ in die gewünschte Gruppe $R_3$ überführt, erforderlichenfalls ein verfahrensgemäss erhältliches Isomerengemisch in die Komponenten auftrennt und das Isomere der Formel I isoliert und gewünschtenfalls eine verfahrensgemäss erhältliche Verbindung in eine andere Verbindung der Formel I umwandelt und/oder eine verfahrensgemäss erhältliche freie Verbindung in ein Salz oder ein verfahrensgemäss erhältliches Salz in die freie Verbindung oder in ein anderes Salz überführt.

2. Verfahren gemäss Anspruch 1, dadurch gekennzeichnet, dass man Verbindungen der Formel I, worin die Reste $R_1$ und $R_2$ unabhängig voneinander unsubstituierte oder durch Niederalkyl, Niederalkoxy bzw. an vicinalen C-Atomen Niederalkyl(id)endioxy, Hydroxy, Halogen, Niederalkanoyloxy, Niederalkylthio, Niederalkansulfinyl, Niederalkansulfonyl, Amino, Mono- oder Diniederalkylamino und/oder Trifluormethyl substituierte 6 bis und mit 10 C-Atomen aufweisende Aryl- bzw. monocyclische, ein Sauerstoff- oder Schwefelatom und gegebenenfalls zusätzlich ein Stickstoffatom aufweisende 5-gliedrige bzw. gegebenenfalls N-oxidierte ein oder zwei Stickstoffatom(e) aufweisende 6-gliedrige Heteroarylreste bedeuten, n für 0, 1 oder 2 steht und $R_3$ Carboxyniederalkyl, Niederalkoxycarbonylniederalkyl, Carbamylniederalkyl, N-Mono- und N,N-Diniederalkylcarbamylniederalkyl, Cyanoniederalkyl oder die Hydroxy-, Niederalkanoyloxy-, Niederalkoxy-, Niederalkylendioxy-, Niederalkylthio-, Niederalkylendithio, Niederalkansulfinyl-, Niederalkansulfonyl- bzw. Oxogruppe(n) in höherer als der $\alpha$-Stellung tragendes Mono- oder Dihydroxyniederalkyl, Niederalkanoyloxyniederalkyl, Mono- oder Diniederalkoxyniederalkyl, Niederalkylendioxyniederalkyl, Niederalkylendithioniederalkyl, Mono- oder Diniederalkylthioniederalkyl, Niederalkansulfinylniederalkyl, Niederalkansulfonylniederalkyl oder Oxoniederalkyl bedeutet, oder deren Salze herstellt.

3. Verfahren gemäss Anspruch 1, dadurch gekennzeichnet, dass man Verbindungen der Formel I, worin die Reste $R_1$ und $R_2$ unabhängig voneinander unsubstituiertes oder durch Niederalkyl, Niederalkoxy, Hydroxy, Halogen, Niederalkanoyloxy, Niederalkylthio, Niederalkansulfonyl, Diniederalkylamino und/oder Trifluormethyl substituiertes Phenyl bzw. unsubstituiertes oder durch Niederalkyl, Niederalkoxy, Halogen oder Hydroxy substituiertes Furyl, Thienyl, Pyridyl bzw. 1-Oxidopyridyl,

bedeuten, n für 0, 1 oder 2 steht und $R_3$ Carboxyniederalkyl, Niederalkoxycarbonylniederalkyl, Carbamylniederalkyl, N-Mono- oder N,N-Diniederalkylcarbamylniederalkyl, oder die Hydroxy-, Niederalkoxy-, Niederalkylendioxy-, Niederalkylidendioxy- bzw. Oxogruppe(n) in höherer als der α-Stellung tragendes Hydroxyniederalkyl, Mono- oder Diniederalkoxyniederalkyl, Niederalkylendioxyniederalkyl, Niederalkylidendioxyniederalkyl oder Oxoniederalkyl bedeutet, oder deren Salze herstellt.

4. Verfahren gemäss Anspruch 1, dadurch gekennzeichnet, dass man Verbindungen der Formel I, worin $R_1$ und $R_2$ durch Niederalkoxy mit bis und mit 4 C-Atomen substituierte Phenylreste bedeuten, n für 0, 1 oder 2 steht und $R_3$ die Carboxy oder Niederalkoxycarbonylgruppe in α-Stellung tragendes Carboxy- bzw. Niederalkoxycarbonylniederalkyl mit bis und mit 4 C-Atomen im Niederalkyl- und gegebenenfalls Niederalkoxyteil oder die Hydroxy-, Oxo-, Niederalkoxy- bzw. Niederalkylendioxygruppe in höherer als der α-Stellung tragendes Hydroxyniederalkyl mit 2 bis und mit 4 C-Atomen, Oxoniederalkyl mit 2 bis und mit 4 C-Atomen oder Mono- oder Diniederalkoxyniederalkyl bzw. Niederalkylendioxyniederalkyl mit jeweils bis und mit 4 C-Atomen in jedem Alkyl(en)teil bedeutet, oder deren Salze herstellt.

5. Verfahren gemäss Anspruch 1, dadurch gekennzeichnet, dass man Verbindungen der Formel I, worin $R_1$ und $R_2$ durch Niederalkoxy mit bis und mit 4 C-Atomen substituierte Phenylreste bedeuten, n für 0, 1 oder 2 steht und $R_3$ die Carboxy oder Niederalkoxycarbonylgruppe in α-Stellung tragendes Carboxy- bzw. Niederalkoxycarbonylniederalkyl mit bis und mit 4 C-Atomen im Niederalkyl- und gegebenenfalls Niederalkoxyteil bedeutet, oder deren Salze herstellt.

6. Verfahren gemäss Anspruch 1, dadurch gekennzeichnet, dass man 1,5-Bis(p-methoxyphenyl)-3-(2,2-dimethoxyäthylthio)-1H-1,2,4-triazol oder ein Salz davon herstellt.

7. Verfahren gemäss Anspruch 1, dadurch gekennzeichnet, dass man 1,5-Bis(p-methoxyphenyl)-3-methoxycarbonylmethylthio-1H-1,2,4-triazol oder ein Salz davon herstellt.

8. Verfahren gemäss Anspruch 1, dadurch gekennzeichnet, dass man 1,5-Bis(p-methoxyphenyl)-3-(2-oxoäthylthio)-1H-1,2,4-triazol oder ein Salz davon herstellt.

9. Verfahren gemäss Anspruch 1, dadurch gekennzeichnet, dass man 1,5-Bis(p-methoxyphenyl)-3-carboxymethylthio-1H-1,2,4-triazol oder ein Salz davon herstellt.

10. Verfahren gemäss Anspruch 1, dadurch gekennzeichnet, dass man 1,5-Bis(p-methoxyphenyl)-3-(2-carboxyäthylthio)-1H-1,2,4-triazol, 1,5-Bis(p-methoxyphenyl)-3-(3,3-äthylendioxypropylthio)-1H-1,2,4-triazol, 1,5-Bis(p-methoxyphenyl)-3-(3-oxopropylthio)-1H-1,2,4-triazol, 1,5-Bis(p-methoxyphenyl)-3-N,N-dimethylcarbamoylmethylthio-1H-1,2,4-triazol, 1,5-Bis(p-methoxyphenyl)-3-methoxycarbonylmethansulfonyl-1H-1,2,4-triazol, 1,5-Bis(p-methoxyphenyl)-3-methoxycarbonylmethansulfinyl-1H-1,2,4-triazol, 1,5-Bis(p-methoxyphenyl-3-carbamylmethylthio-1,2,4-triazol, 1,5-Bis(p-methoxyphenyl)-3- carboxymethynsulfinyl-1H-1,2,4-triazol, 1,5-Bis(p-methoxyphenyl)-3-cyanomethylthio-1H-1,2,4-triazol, 1,5-Bis(p-chlorphenyl)-3-(2,2-dimethoxyäthylthio)-1H-1,2,4-triazol, 1,5-Bis(p-chlorphenyl)-3-(2-oxoäthylthio)-1H-1,2,4-triazol, 1-(3-Pyridyl)-5-phenyl-3-(2-Hydroxyäthylthio)-1H-1,2,4-triazol, 1,5-Bis(p-methoxyphenyl)-3-carboxymethansulfonyl-1H-1,2,4-triazol, 1,5-Bis(p-methoxyphenyl)-3-(1-äthoxycarbonyläthylthio)-1H,1,2,4-triazol, 1,5-Bis(p-methoxyphenyl)-3-(1-carboxyäthylthio)-1H-1,2,4-triazol, 1,5-Bis(p-methoxyphenyl)-3-carbonylmethylthio-1H-1,2,4-triazol, 1,5-Bis(p-methoxyphenyl)-3-(2-hydroxyäthylthio)-1H-1,2,4-triazol oder jeweils ein Salz davon herstellt.

11. Das Verfahren der Beispiele 1-19 und verfahrensgemäss verwendete neue Ausgangsstoffe, gebildete neue Zwischenprodukte und erhältliche Endprodukte bzw. Verfahren zu ihrer Herstellung.

12. Verfahren zur Herstellung pharmazeutischer Präparate, dadurch gekennzeichnet, dass man eine gemäss einem der Ansprüche 1-10 erhältliche Verbindung mit üblichen pharmazeutischen Hilfs- und/oder Trägerstoffen vermischt.

FO 7.4 KVB/gb*

Europäisches Patentamt

# EUROPÄISCHER RECHERCHENBERICHT

0157259

Nummer der Anmeldung

EP 85 10 3010

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 013 732 (SCHERING AG)<br>* Ansprüche 1-5,42-44 * | 1,11, 12,14, 15 | C 07 D 249/12<br>C 07 D 401/04<br>A 61 K 31/41 |
| A | DE-A-2 558 951 (CANON K.K.)<br>* Tabelle 2, Seite 19, erste Strukturformel von oben * | 1 | |
| A | CHEMICAL ABSTRACTS, Band 93, Nr. 3, 21. Juli 1980, Columbus, Ohio, USA; M. AUGUSTIN et al. "Reactions with N acylimino dithiocarbonic acid diesters"; Seite 693, Zusammenfassungsnr. 26307a; & J. Prakt. Chem., Band 322, Nr. 1, 1980, Seiten 5 5-68 * Formel IV * | 1 | |
| | | | RECHERCHIERTE SACHGEBIETE (Int. Cl.4) |
| A | DE-A-2 805 167 (E.I. DU PONT DE NEMOURS AND CO.)<br>* Anspruch 1 * | 1 | C 07 D 249/00<br>C 07 D 401/00 |

Der vorliegende Recherchenbericht wurde für alle Patentansprüche erstellt.

| Recherchenort | Abschlußdatum der Recherche | Prüfer |
|---|---|---|
| BERLIN | 12-06-1985 | HASS C V F |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument